(19) Europäisches Patentamt
European Patent Office
Office européen des brevets

(11) Veröffentlichungsnummer: **0 253 321**
**A2**

(12) # EUROPÄISCHE PATENTANMELDUNG

(21) Anmeldenummer: **87109962.8**

(22) Anmeldetag: **10.07.87**

(51) Int. Cl.⁴: **C07C 143/79**, C07D 237/04, A61K 31/18, A61K 31/50

(30) Priorität: **16.07.86 DE 3623944**

(43) Veröffentlichungstag der Anmeldung: **20.01.88 Patentblatt 88/03**

(84) Benannte Vertragsstaaten: **AT BE CH DE ES FR GB GR IT LI LU NL SE**

(71) Anmelder: **Dr. Karl Thomae GmbH**
**Postfach 1755**
**D-7950 Biberach (Riss)(DE)**

(72) Erfinder: **Nickl, Josef, Dr., Dipl.-Chem.**
**Silcherstrasse 8**
**D-7950 Biberach 1(DE)**
Erfinder: **Heckel, Armin, Dr., Dipl.-Chem.**
**Lamparterweg 1**
**D-7950 Biberach 1(DE)**
Erfinder: **Müller, Erich, Dr., Dipl.-Chem.**
**Talfeldstrasse 34**
**D-7950 Biberach 1(DE)**
Erfinder: **Narr, Berthold, Dr., Dipl.-Chem.**
**Obere Au 5**
**D-7950 Biberach 1(DE)**
Erfinder: **Weisenberger, Johannes, Dr., Dipl.-Chem.**
**Haydnweg 5**
**D-7950 Biberach 1(DE)**
Erfinder: **Eisert, Wolfgang, Prof. Dr.**
**Friedrich Goll Weg 5**
**D-7950 Biberach 1(DE)**
Erfinder: **Müller, Thomas, Dr., Dipl.-Chem.**
**Gymnasiumstrasse 165**
**D-7950 Biberach 1(DE)**

(54) **Neue Benzolsulfonamido-indanylverbindungen, diese Verbindungen enthaltende Arzneimittel und Verfahren zu ihrer Herstellung.**

(57) Die Erfindung betrifft neue Benzolsulfonamido-indanylverbindungen der allgemeinen Formel

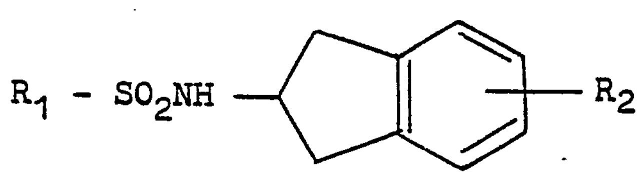

(I)

in der
$R_1$ eine gegebenenfalls durch ein Halogenatom, eine Methyl-oder Methoxygruppe mono-oder disubstituierte Phenylgruppe, wobei die Substituenten gleich oder verschieden sein können, und
$R_2$ eine gegebenenfalls über eine geradkettige oder verzweigte Alkylengruppe oder über eine Alkenylen-

EP 0 253 321 A2

gruppe gebundene Hydroxycarbonyl-oder Alkoxycarbonylgruppe, wobei jeweils eine Methylengruppe der vorstehend erwähnten Alkylen-oder Alkenylenreste, welche mit dem Indanylrest verknüpft sein muß, durch eine Hydroxymethylen-oder Carbonylgruppe ersetzt sein kann und gleichzeitig die Methylengruppe, über die die vorstehend erwähnte Alkoxycarbonylgruppe gebunden ist, durch eine weitere Alkoxycarbonylgruppe substituiert sein kann, oder eine 4,5-Dihydro-pyridazin-3(2H)-on-6-yl-oder Pyridazin-3(2H)-on-6-yl-gruppe, welche in 4-oder 5-Stellung durch eine Alkylgruppe und/oder in 4-Stellung durch eine Alkoxycarbonylgruppe substituiert sein können bedeuten, deren Enantiomere und deren Salze mit anorganischen oder organischen Basen, falls $R_2$ eine Hydroxycarbonylgruppe enthält.

Die neuen Verbindungen weisen wertvolle pharmakologische Eigenschaften auf, insbesondere antithrombotische Wirkungen, außerdem stellen diese Thromboxanantagonisten dar, und lassen sich nach an und für sich bekannten Verfahren herstellen.

**Neue Benzolsulfonamido-indanylverbindungen, diese Verbindungen enthaltende Arzneimittel und Verfahren zu ihrer Herstellung**

Gegenstand der vorliegenden Erfindung sind neue Benzolsulfonamido-indanylverbindungen der allgemeinen Formel

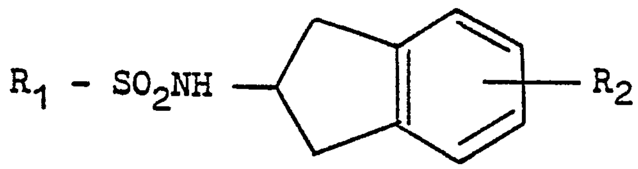

(I)

deren Enantiomere und deren Additionssalze, insbesondere für die pharmazeutische Anwendung deren physiologisch verträgliche Additionssalze mit anorganischen oder organischen Basen, falls $R_2$ eine Hydroxycarbonylgruppe darstellt oder enthält, welche wertvolle pharmakologische Eigenschaften aufweisen, insbesondere antithrombotische Wirkungen, außerdem stellen die neuen Verbindungen Thromboxanantagonisten dar.

Gegenstand der vorliegenden Erfindung sind somit die neuen Verbindungen der obigen allgemeinen Formel I, deren Additionssalze mit anorganischen oder organischen Basen, insbesondere für die pharmazeutische Verwendung deren physiologisch verträgliche Additionssalze, diese Verbindungen enthaltende Arzneimittel und Verfahren zu ihrer Herstellung.

In der obigen allgemeinen Formel bedeutet

$R_1$ eine gegebenenfalls durch ein Halogenatom, eine Methyl-oder Methoxygruppe mono-oder disubstituierte Phenylgruppe, wobei die Substituenten gleich oder verschieden sein können, und

$R_2$ eine gegebenenfalls über eine geradkettige oder verzweigte Alkylengruppe mit 1 bis 5 Kohlenstoffatomen oder über eine Alkenylengruppe mit 2 bis 5 Kohlenstoffatomen gebundene Hydroxycarbonyl-oder Alkoxycarbonylgruppe mit insgesamt 2 bis 4 Kohlenstoffatomen, wobei jeweils eine Methylengruppe der vorstehend erwähnten Alkylen-oder Alkenylenreste, welche mit dem Indanylrest verknüpft sein muß, durch eine Hydroxymethylen-oder Carbonylgruppe ersetzt sein kann und gleichzeitig die Methylengruppe, über die die vorstehend erwähnte Alkoxycarbonylgruppe gebunden ist, durch eine weitere Alkoxycarbonylgruppe mit insgesamt 2 bis 4 Kohlenstoffatomen substituiert sein kann, oder eine 4,5-Dihydro-pyridazin-3(2H)-on-6-yl-oder Pyridazin-3(2H)-on-6-yl-gruppe, welche in 4-oder 5-Stellung durch eine Alkylgruppe mit 1 bis 3 Kohlenstoffatomen und/oder in 4-Stellung durch eine Alkoxycarbonylgruppe mit insgesamt 2 bis 4 Kohlenstoffatomen substituiert sein können.

Für die bei der Definition der Reste $R_1$ und $R_2$ eingangs erwähnten Bedeutungen kommt beispielsweise für $R_1$ die der Phenyl-, 2-Methylphenyl-, 3-Methylphenyl-, 4-Methylphenyl-, 2-Methoxyphenyl-, 3-Methoxyphenyl-, 4-Methoxyphenyl-, 2-Fluorphenyl-, 3-Fluorphenyl-, 4-Fluorphenyl-, 2-Chlorphenyl-, 3-Chlorphenyl-, 4-Chlorphenyl-, 2-Bromphenyl-, 4-Bromphenyl-, 3,4-Dimethylphenyl-, 3,4-Dimethoxyphenyl-, 2,4-Difluorphenyl-, 2,4-Dichlorphenyl-, 2,5-Dichlorhenyl-, 2,4-Dibromphenyl-oder Methyl-chlorphenylgruppe und

für $R_2$ die der Hydroxycarbonyl-, Hydroxycarbonylmethyl-, 1-Hydroxycarbonyl-äthyl-, 2-Hydroxycarbonyl-äthyl-, 1-Hydroxycarbonyl-propyl-, 3-Hydroxycarbonyl-propyl-, 1-Hydroxycarbonyl-butyl-, 4-Hydroxycarbonyl-butyl-, 1-Hydroxycarbonyl-1-methyl-äthyl-, 2-Hydroxycarbonyl-1-methyl-äthyl-, 2-Hydroxycarbonyl-äthenyl-, 2-Hydroxycarbonyl-1-methyl-äthenyl-, 3-Hydroxycarbonyl-propenyl-, 2-Hydroxycarbonyl-äthanon-(1)-yl-, 3-Hydroxycarbonyl-n-propanon-(1)-yl-, 4-Hydroxycarbonyl-n-butanon-(1)-yl-, 5-Hydroxycarbonyl-n-pentanon-(1)-yl-, 2-Hydroxycarbonyl-2-methyl-äthanon-(1)-yl-, 3-Hydroxycarbonyl-2-methyl-n-propanon-(1)-yl-, 3-Hydroxycarbonyl-3-methyl-n-propanon-(1)-yl-, 4-Hydroxycarbonyl-2-methyl-n-butanon-(1)-yl-, 4-Hydroxycarbonyl-3-methyl-n-butanon-(1)-yl-, 4-Hydroxycarbonyl-4-methyl-n-butanon-(1)-yl-, 2-Hydroxycarbonyl-2-äthyläthanon-(1)-yl-, 2-Hydroxycarbonyl-2-n-propyläthanon-(1)-yl-, 2-Hydroxycarbonyl-2-äthyl-n-propanon-(1)-yl-, 3-Hydroxycarbonyl-3-äthyl-n-propanon-(1)-yl-, 4-Hydroxycarbonyl-n-buten-2-on-(1)-yl-, 5-Hydroxycarbonyl-n-penten-2-on-(1)-yl-, 2-Hydroxycarbonyl-1-hydroxy-äthyl-, 3-Hydroxycarbonyl-1-hydroxy-n-propyl-, 4-Hydroxycarbonyl-1-hydroxy-n-butyl-, 5-Hydroxycarbonyl-1-hydroxy-n-pentyl-, 2-Hydroxycarbonyl-2-methyl-1-hydroxy-äthyl-, 2-Hydroxycarbonyl-2-äthyl-1-hydroxy-äthyl-, 2-Hydroxycarbonyl-2-isopropyl-1-hydroxy-äthyl-, 3-Hydroxycarbonyl-2-methyl-1-hydroxy-n-propyl-, 3-Hydroxycarbonyl-2-äthyl-1-hydroxy-n-propyl-, 3-Hydroxycarbonyl-3-methyl-1-hydroxy-n-propyl-, 3-Hydroxycarbonyl-3-äthyl-1-hydroxy-n-propyl-, 4-Hydroxycarbonyl-2-methyl-1-hydroxy-n-butyl-,

4-Hydroxycarbonyl-3-methyl-1-hydroxy-n-butyl-, 4-Hydroxycarbonyl-4-methyl-1-hydroxy-n-butyl-, Methoxycarbonyl-, Äthoxycarbonyl-, n-Propoxycarbonyl-, Methoxycarbonylmethyl-, Äthoxycarbonylmethyl-, Isopropoxycarbonylmethyl-, 2-Methoxycarbonyl-äthyl-, 2-Äthoxycarbonyl-äthyl-, 3-Methoxycarbonyl-propyl-, 4-Äthoxycarbonyl-butyl-, 2-Methoxycarbonyl-1-methyl-äthyl-, 2-Äthoxycarbonyl-1-methyl-äthyl-, 2-Isopropoxycarbonyl-1-methyl-äthyl-, 2-Methoxycarbonyl-äthenyl-, 2-Methoxycarbonyl-1-methyläthenyl-, 2-Äthoxycarbonyl-1-methyl-äthenyl-, 3-Methoxycarbonyl-propenyl-, 2-Methoxycarbonyl-äthanon-(1)-yl-, 2-(2-Methoxyäthoxycarbonyl)-äthanon-(1)-yl-, 3-Methoxycarbonyl-n-propanon-(1)-yl-, 3-Äthoxycarbonyl-n-propanon-(1)-yl-, 3-(2-Äthoxy-äthoxycarbonyl)-n-propanon(1)-yl-, 3-(3-Methoxy-n-propoxy-carbonyl)-n-propanon-(1)-yl-, 3-n-Propoxycarbonyl-n-propanon-(1)-yl-, 4-Äthoxycarbonyl-n-butanon-(1)-yl-, 5-Äthoxycarbonyl-n-pentanon-(1)-yl-, 2-Äthoxycarbonyl-2-methyl-äthanon-(1)-yl-, 3-Äthoxycarbonyl-2-methyl-n-propanon-(1)-yl-, 3-Äthoxycarbonyl-3-methyl-n-propanon-(1)-yl-, 4-Äthoxycarbonyl-2-methyl-n-butanon-(1)-yl-, 4-Äthoxycarbonyl-3-methyl-n-butanon-(1)-yl-, 4-Äthoxycarbonyl-4-methyl-n-butanon-(1)-yl-, 2-Äthoxycarbonyl-2-äthyl-äthanon-(1)-yl-, 2-Äthoxycarbonyl-2-n-propyl-äthanon-(1)-yl-, 3-Äthoxycarbonyl-2-äthyl-n-propanon-(1)-yl-, 3-Äthoxycarbonyl-3-äthyl-n-propanon-(1)-yl-, 3-Äthoxycarbonyl-n-propanon-(1)-yl-, 3-Äthoxycarbonyl-2-methyl-n-propanon-(1)-yl-, 3-Äthoxycarbonyl-3-methyl-n-propanon-(1)-yl-, 4-Äthoxycarbonyl-n-buten-2-on-(1)-yl-, 5-Äthoxycarbonyl-n-penten-2-on-(1)-yl-, Äthoxycarbonyl-hydroxymethyl-, 2-Äthoxycarbonyl-1-hydroxyäthyl-, 3-Äthoxycarbonyl-1-hydroxy-n-propyl-, 4-Äthoxycarbonyl-1-hydroxy-n-butyl-, 5-Äthoxycarbonyl-1-hydroxy-n-pentyl-, 2-Äthoxycarbonyl-2-methyl-1-hydroxy-äthyl-, 2-Äthoxycarbonyl-2-äthyl-1-hydroxy-äthyl-, 2-Äthoxycarbonyl-2-isopropyl-1-hydroxy-äthyl-, 3-Äthoxycarbonyl-2-methyl-1-hydroxy-n-propyl-, 3-Äthoxycarbonyl-2-äthyl-1-hydroxy-n-propyl-, 3-Äthoxycarbonyl-3-methyl-1-hydroxy-n-propyl-, 3-Äthoxycarbonyl-3-äthyl-1-hydroxy-n-propyl-, 4-Äthoxycarbonyl-2-methyl-1-hydroxy-n-butyl-, 4-Äthoxycarbonyl-3-methyl-1-hydroxy-n-butyl-, 4-Äthoxycarbonyl-4-methyl-1-hydroxy-n-butyl-, 4,5-Dihydro-pyridazin-3(2H)-on-6-yl-, 4,5-Dihydro-5-methyl-pyridazin-3(2H)-on-6-yl-, 4,5-Dihydro-5-äthyl-pyridazin-3(2H)-on-6-yl-, 4,5-Dihydro-5-n-propyl-pyridazin-3-(2H)-on-6-yl-, Pyridazin-3(2H)-on-6-yl-, 5-Methyl-pyridazin-3(2H)-on-6-yl-, 5-Äthyl-pyridazin-3(2H)-on-6-yl-, 5-n-Propyl-pyridazin-3(2H)-on-6-yl-, 5-Isopropyl-pyridazin-3(2H)-on-6-yl-, 4,5-Dihydro-4-methoxycarbonyl-pyridazin-3(2H)-on-6-yl-, 4,5-Dihydro-4-äthoxycarbonyl-pyri dazin-3(2H)-on-6-yl-, 4,5-Dihydro-4-n-propoxycarbonyl-pyridazin-3(2H)-on-6-yl-, 4,5-Dihydro-4-isopropoxycarbonyl-pyridazin-3(2H)-on-6-yl-, 4,5-Dihydro-4-methoxycarbonyl-5-methyl-pyridazin-3(2H)-on-6-yl-, 4,5-Dihydro-4-äthoxycarbonyl-5-methyl-pyridazin-3(2H)-on-6-yl-, 4,5-Dihydro-4-n-propoxycarbonyl-5-methyl-pyridazin-3(2H)-on-6-yl-, 4,5-Dihydro-4-äthoxycarbonyl-5-äthyl-pyridazin-3(2H)-on-6-yl-, 4,5-Dihydro-4-äthoxycarbonyl-5-n-propyl-pyridazin-3(2H)-on-6-yl-, 4-Äthoxycarbonyl-pyridazin-3(2H)-on-6-yl-, 5-Methyl-4-äthoxycarbonyl-pyridazin-3(2H)-on-6-yl-, 5-Äthyl-4-äthoxycarbonyl-pyridazin-3(2H)-on-6-yl-, 5-n-Propyl-4-äthoxycarbonyl-pyridazin-3(2H)-on-6-yl-oder 5-Isopropyl-4-äthoxycarbonyl-pyridazin-3(2H)-on-6-yl-gruppe in Betracht.

Bevorzugte Verbindungen der obigen allgemeinen Formel I sind diejenigen, in denen

$R_1$ eine gegebenenfalls durch eine Methyl-oder Methoxygruppe substituierte Phenylgruppe oder eine durch ein Fluor-, Chlor-oder Bromatom mono-oder disubstituierte Phenylgruppe und

$R_2$ eine gegebenenfalls über eine geradkettige oder verzweigte Alkylengruppe mit 1 bis 4 Kohlenstoffatomen oder über eine geradkettige oder verzweigte Alkenylengruppe mit 2 bis 4 Kohlenstoffatomen gebundene Hydroxycarbonyl-oder Alkoxycarbonylgruppe mit insgesamt 2 oder 3 Kohlenstoffatomen, wobei jeweils eine Methylengruppe der vorstehend erwähnten Alkylen-oder Alkenylengruppen, welche mit dem Indanylrest verknüpft sein muß, durch eine Hydroxymethylen-oder Carbonylgruppe ersetzt sein kann, oder eine gegebenenfalls in 5-Stellung durch eine Methylgruppe substituierte 4,5-Dihydro-pyridazin-3(2H)-on-6-yl-oder Pyridazin-3(2H)-on-6-yl-gruppe, welche zusätzlich in 4-Stellung durch eine Alkoxycarbonylgruppe mit insgesamt 2 oder 3 Kohlenstoffatomen substituiert sein kann, bedeuten.

Besonders bevorzugte Verbindungen der obigen allgemeinen Formel I sind jedoch diejenigen, in denen

$R_1$ eine gegebenenfalls durch ein Fluor-oder Chloratom, durch eine Methyl-oder Methoxygruppe substituierte Phenylgruppe und

$R_2$ eine Hydroxycarbonylmethyl-, 4-Hydroxycarbonyl-n-propyl-, 3-Hydroxycarbonyl-n-propanon-(1)-yl-, 4,5-Dihydro-pyridazin-3(2H)-on-6-yl-oder 4,5-Dihydro-5-methyl-pyridazin-3(2H)-on-yl-gruppe bedeuten.

Erfindungsgemäß erhält man die neuen Verbindungen nach folgenden Verfahren:

a.) Acylierung einer Verbindung der allgemeinen Formel

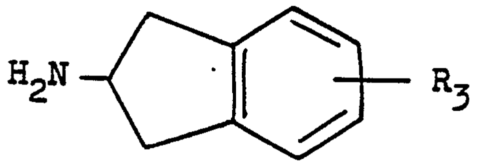 (II)

in der

$R_3$ die für $R_2$ eingangs erwähnten Bedeutungen aufweist, wobei jedoch eine Hydroxygruppe im Rest $R_2$ durch eine hydrolytisch oder hydrogenolytisch abspaltbare Schutzgruppe wie eine Alkoxy-oder Benzyloxygruppe geschützt sein kann, mit einem Phenylsulfonsäurederivat der allgemeinen Formel

$R_1$ -$SO_2X$ ,(III)

in der

$R_1$ wie eingangs definiert ist und

X eine nucleophile Austrittsgruppe wie ein Halogenatom oder eine Alkoxygruppe, z.B. ein Chlor-oder Bromatom, eine Methoxy-oder Äthoxygruppe, darstellt und gegebenenfalls anschließende Abspaltung eines verwendeten Schutzrestes.

Die Umsetzung wird vorzugsweise in einem Lösungsmittel wie Methanol, Äthanol, Wasser/Methanol, Dioxan, Tetrahydrofuran oder Chloroform gegebenenfalls in Gegenwart eines säurebindenden Mittels wie Kaliumcarbonat, Triäthylamin oder Pyridin, wobei die beiden letzteren auch als Lösungsmittel verwendet werden können, zweckmäßigerweise bei Temperaturen zwischen 0 und 50°C, vorzugsweise jedoch bei Raumtemperatur, durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

b.) Zur Herstellung von Verbindungen der allgemeinen Formel I, in der $R_2$ eine Hydroxycarbonylgruppe darstellt oder enthält:

Abspaltung eines Schutzrestes von einer Verbindung der allgemeinen Formel

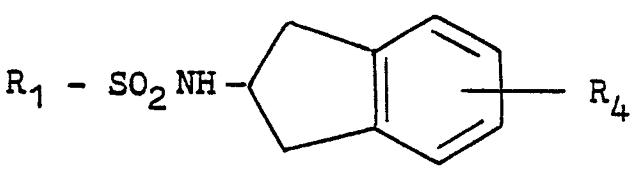 (IV)

in der

$R_1$ wie eingangs definiert ist und

$R_4$ die für $R_2$ eingangs erwähnten Bedeutungen besitzt, wobei jedoch die Carboxygruppe durch einen hydrolytisch, thermolytisch oder hydrogenolytisch abspaltbare Schutzgruppe geschützt ist oder ein funktionelles Derivat der Carboxygruppe darstellt und/oder der Rest $R_2$ eine durch einen Schutzrest geschützte Hydroxygruppe enthält.

Als hydrolysierbare Gruppen kommen beispielsweise funktionelle Derivate der Carboxygruppe wie deren unsubstituierte oder substituierte Amide, Ester, Thioester, Orthoester, Iminoäther, Amidine oder Anhydride, die Nitrilgruppe, Äthergruppen wie die Methoxy-oder Benzyloxygruppe oder Lactone und als thermolytisch abspaltbare Gruppen beispielsweise Ester mit tertiären Alkoholen, z.B. der tert.Butylester, und als hydrogenolytisch abspaltbare Gruppen beispielsweise Aralkylgruppen, z.B. die Benzylgruppe, in Betracht.

Die Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.

Enthält beispielsweise eine Verbindung der allgemeinen Formel IV eine Nitril-oder Aminocarbonylgruppe, so können diese Gruppen vorzugsweise mittels 100%iger Phosphorsäure bei Temperaturen zwischen 100 und 180°C, vorzugsweise bei Temperaturen zwischen 120 und 160°C, oder auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen 0 und 50°C in die Carboxygruppe übergeführt werden.

Enthält beispielsweise eine Verbindung der allgemeinen Formel IV die tert.Butyloxycarbonylgruppe, so kann die tert.Butylgruppe auch thermisch gegebenenfalls in einem inerten Lösungsmittel wie Methlenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40 und 100°C, abgespalten werden.

Enthält beispielsweise eine Verbindung der allgemeinen Formel IV die Benzyloxy-oder Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Methanol/Wasser, Äthanol/Wasser, Eisessig, Essigsäureäthylester, Dioxan oder Dimethylformamid vorzugsweise bei Temperaturen zwischen 0 und 50°C, z.B. bei Raumtemperatur und einem Wasserstoffdruck von 1 bis 5 bar abgespalten werden. Bei der Hydrogenolyse kann gleichzeitig eine halogenhaltige Verbindung enthalogeniert und eine vorhandende Doppelbindung aufhydriert werden.

c.) Zur Herstellung von Verbindungen der allgemeinen Formel I, in der $R_2$ benachbart zum Indanylrest eine Carbonylgruppe enthält:

Acylierung einer Verbindung der allgemeinen Formel

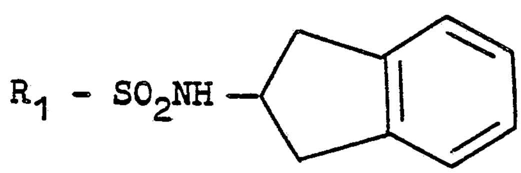

(V)

in der

$R_1$ wie eingangs definiert ist, mit einer Verbindung der allgemeinen Formel

$Y - R_5$ ,(VI)

in der

$R_5$ die für $R_2$ eingangs erwähnten Bedeutungen besitzt, wobei jedoch $R_2$ benachbart zu Y eine Carbonylgruppe enthalten muß und gleichzeitig eine gegebenenfalls vorhandene Hydroxycarbonylgruppe durch einen hydrolytisch oder hydrogenolytisch abspaltbaren Schutzrest wie eine Alkoxy-oder Benzylgruppe geschützt sein kann, und

Y eine nucleophile Austrittsgruppe wie ein Halogenatom, z.B. ein Chlor-, Brom-oder Jodatom, darstellen, oder dessen Anhydrid in Gegenwart einer Lewis-Säure und gegebenenfalls anschließende Abspaltung eines verwendeten Schutzrestes.

Die Friedel-Craft's-Acylierung wird vorzugsweise in einem Lösungsmittel wie Äthylenchlorid oder Nitrobenzol in Gegenwart einer Lewis-Säure wie Aluminiumchlorid, Bortrifluorid oder Zinkchlorid zweckmäßigerweise bei Temperaturen zwischen 0 und 50°C, vorzugsweise jedoch bei Raumtemperatur, durchgeführt.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser Tetrahydrofuran/Wasser oder Dioxan/Wasser, in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators

wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

d.) Zur Herstellung von Verbindungen der allgemeinen Formel I, in der $R_2$ benachbart zum Indanylrest eine Hydroxymethylen-oder Methylengruppe enthält:
Reduktion einer Verbindung der allgemeinen Formel

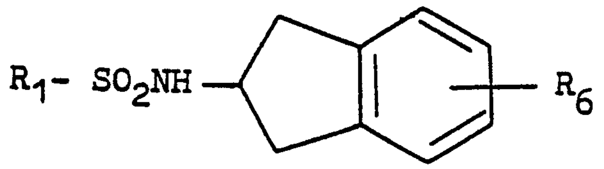

(VII)

in der
$R_1$ wie eingangs definiert ist und
$R_6$ die für $R_2$ eingangs erwähnten Bedeutungen aufweist, wobei jedoch $R_2$ benachbart zum Indanylrest eine Carbonylgruppe enthalten muß.

Die Reduktion wird in einem geeigneten Lösungsmittel wie Methanol, Äthanol, Äther, Tetrahydrofuran, Dioxan oder Eisessig in Gegenwart von katalytisch angeregtem Wasserstoff, z.B. von Wasserstoff in Gegenwart von Platin oder Palladium/Kohle, und gegebenenfalls in Gegenwart einer Säure wie Salzsäure oder Perchlorsäure oder in Gegenwart eines Metallhydrids wie Natrium-borhydrid, Lithium-borhydrid oder Lithium-aluminiumhydrid bei Temperaturen zwischen 0 und 100°C, vorzugsweise bei Temperaturen zwischen 20 und 80°C, durchgeführt.

Zur Herstellung einer Verbindung der allgemeinen Formel I, in der $R_2$ eine Hydroxymethylengruppe enthält, wird die Umsetzung vorzugsweise mit Natrium-borhydrid in Methanol und bei Raumtemperatur durchgeführt.

Enthält eine Verbindung der allgemeinen Formel VII im Rest $R_6$ eine Doppelbindung, so kann diese bei der Umsetzung mit Wasserstoff in Gegenwart von katalytisch angeregtem Wasserstoff gleichzeitig aufhydriert werden.

e.) Zur Herstellung von Verbindungen der allgemeinen Formel I, in der $R_2$ einen der eingangs erwähnten gesättigten Reste darstellt:
Hydrierung einer Verbindung der allgemeinen Formel

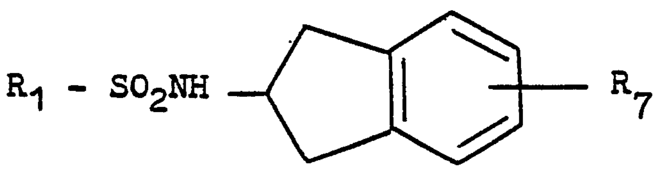

(VIII)

in der
$R_1$ wie eingangs definiert ist und
$R_7$ einen der für $R_2$ eingangs erwähnten ungesättigen Rest darstellt.

Die Hydrierung wird in einem geeigneten Lösungsmittel wie Methanol, Äthanol, Dioxan, Essigester oder Eisessig mit katalytisch angeregtem Wasserstoff oder mit nascierendem Wasserstoff bei Temperaturen zwischen 0 und 50°C, vorzugsweise bei Raumtemperatur, durchgeführt. Hierbei kann eine gegebenenfalls im Rest $R_7$ vorhandene Carbonylgruppe gleichzeitig in eine Hydroxymethylengruppe übergeführt werden.

Zur Herstellung einer Verbindung der allgemeinen Formel I, in der $R_2$ eine Carbonylgruppe enthält, wird die Umsetzung vorzugsweise in Gegenwart von Zink/Eisessig und bei Raumtemperatur durchgeführt.

f.) Zur Herstellung von Verbindungen der allgemeinen Formel I, in der $R_2$ einen Pyridazinonring darstellt:
Umsetzung einer Verbindung der allgemeinen Formel

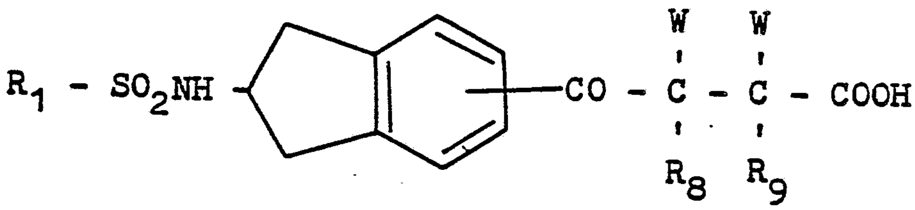 (IX)

in der
$R_1$ wie eingangs definiert ist,
$R_8$ ein Wasserstoffatom oder eine Alkylgruppe mit 1 bis 3 Kohlenstoffatomen,
$R_9$ ein Wasserstoffatom, eine Alkylgruppe mit 1 bis 3 Kohlenstoffatomen oder eine Alkoxycarbonylgruppe mit insgesamt 2 bis 4 Kohlenstoffatomen, wobei jedoch nur einer der Reste $R_8$ oder $R_9$ eine Alkylgruppe mit 1 bis 3 Kohlenstoffatomen darstellen kann, und
W jeweils ein Wasserstoffatom oder zusammen eine weitere Bindung darstellen, oder deren reaktionsfähige Derivate wie deren Ester, Amide oder Halogenide mit Hydrazin.

Die Umsetzung wird zweckmäßigerweise in einem Lösungsmittel wie Methanol, Äthanol, Isopropanol, Eisessig, Propionsäure und/oder in einem Überschuß von Hydrazin bzw. Hydrazin-hydrat bei Temperaturen zwischen 0 und 200°C, z.B. bei Temperaturen zwischen 20 und 150°C, vorzugsweise jedoch bei der Siedetemperatur des Reaktionsgemisches, und gegebenenfalls in Gegenwart einer Säure als Kondensationsmittel wie Schwefelsäure oder p-Toluolsulfonsäure durchgeführt. Die Umsetzung kann jedoch auch ohne Lösungsmittel durchgeführt werden.

g.) Zur Herstellung von Verbindungen der allgemeinen Formel I, in der $R_2$ die Carboxygruppe darstellt:
Umsetzung einer Verbindung der allgemeinen Formel

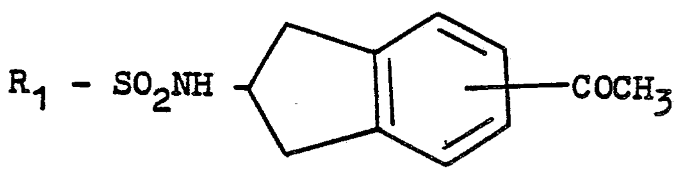 (X)

in der
$R_1$ wie eingangs definiert ist, mit einem Hypohalogenit.

Die Haloformreaktion wird vorzugsweise in einem wässrigen Lösungsmittel wie Wasser/Tetrahydrofuran oder Wasser/Dioxan bei Temperaturen zwischen 0 und 50°C, vorzugsweise jedoch bei Raumtemperatur, durchgeführt. Hierbei wird zweckmäßigerweise das eingesetzte Hypohalogenit, z.B. Natriumhypobromit, im Reaktionsgemisch durch Umsetzung von Brom mit Natronlauge hergestellt.

h.) Zur Herstellung von Verbindungen der allgemeinen Formel I, in der $R_2$ die Hydroxycarbonylmethylgruppe darstellt:
Umsetzung einer Verbindung der allgemeinen Formel

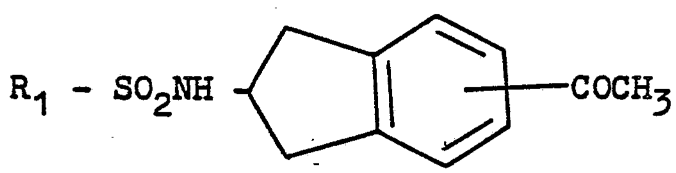 (X)

in der
$R_1$ wie eingangs definiert ist, mit Schwefel in Gegenwart eines Amins und anschließende Verseifung.

Die Willgerodt-Reaktion wird mit Schwefel in Gegenwart eines Amins wie Morpholin bei erhöhten Temperaturen, z.B. bei Temperaturen zwischen 100 und 150°C, durchgeführt. Die an schließende Hydrolyse des so erhaltenen Amids wird in Trichloressigsäure oder in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.

i.) Zur Herstellung von Verbindungen der allgemeinen Formel I, in der $R_2$ eine 2-Hydroxycarbonyl-äthenyl-oder 2-Alkoxycarbonyl-äthenylgruppe darstellt:
Umsetzung einer Verbindung der allgemeinen Formel

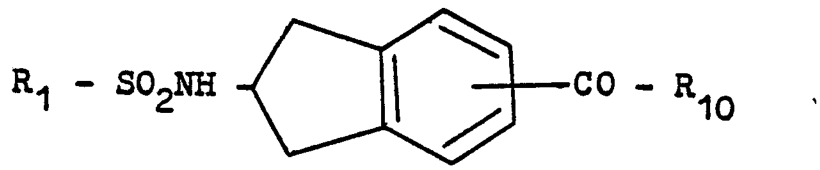

(XI)

in der
$R_1$ wie eingangs definiert ist und
$R_{10}$ ein Wasserstoffatom oder eine Alkylgruppe mit 1 bis 3 Kohlenstoffatomen darstellt, mit einem entsprechenden Phosphonoessigsäure-trialkylester und erforderlichenfalls anschließende Hydrolyse.

Die Umsetzung wird in Gegenwart einer Base wie Kalium-tert.butylat oder Natriumhydrid und in einem Lösungsmittel wie Dimethylformamid bei Temperaturen zwischen 25 und 100°C, vorzugsweise bei Temperaturen zwischen 50 und 75°C, durchgeführt.

Die gegebenenfalls anschließende erforderliche Hydrolyse wird zweckmäßigerweise entweder in Gegenwart einer Säure wie Salzsäure, Schwefelsäure, Phosphorsäure oder Trichloressigsäure oder in Gegenwart einer Base wie Natriumhydroxid oder Kaliumhydroxid in einem geeigneten Lösungsmittel wie Wasser, Wasser/Methanol, Äthanol, Wasser/ Äthanol, Wasser/Isopropanol oder Wasser/Dioxan bei Temperaturen zwischen -10 und 120°C, z.B. bei Temperaturen zwischen Raumtemperatur und der Siedetemperatur des Reaktionsgemisches, durchgeführt.

k) Zur Herstellung von Verbindungen der allgemeinen Formel I, in der $R_2$ einen gegebenenfalls in 4-oder 5-Stellung durch eine Alkylgruppe substituierten 4,5-Dihydro-pyridazin-3-on-oder Pyridazin-3-on-Ring darstellt:
Decarboxylierung einer gegebenenfalls im Reaktionsgemisch gebildeten Verbindung der allgemeinen Formel

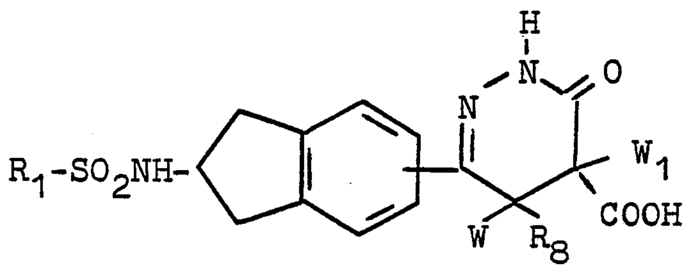

(XII)

in der
$R_1$ wie eingangs definiert ist,
$R_8$ ein Wasserstoffatom oder eine Alkylgruppe mit 1 bis 3 Kohlenstoffatomen,
W ein Wasserstoffatom und
$W_1$ ein Wasserstoffatom oder eine Alkylgruppe mit 1 bis 3 Kohlenstoffatomen oder W und $W_1$ zusammen eine weitere Bindung bedeuten.

Die Decarboxylierung wird zweckmäßigerweise in einem geeigneten Lösungsmittel wie Methanol/Wasser, Äthanol/Wasser oder Dioxan/Wasser und vorzugsweise in Gegenwart einer Säure wie Salzsäure oder Schwefelsäure bei Temperaturen zwischen 0 und 150°C, vorzugsweise bei Temperaturen zwischen 50 und 100°C, durchgeführt. Hierbei wird zweckmäßigerweise ausgehend von einem entsprechenden Carbonsäurederivat der allgemeinen Formel XII, vorzugsweise ausgehend von einem entsprechenden Ester wie dem Methyl-, Ethyl-, Isopropyl-oder Benzylester, eine Verbindung der allgemeinen Formel XII durch Hydrolyse in Gegenwart einer Säure wie Salzsäure oder Schwefelsäure oder in Gegenwart einer Base wie Natriumhydroxid oder Kaliumhydroxid im Reaktionsgemisch hergestellt. Die Decarboxylierung kann jedoch auch ohne Lösungsmittel durch Erhitzen durchgeführt werden.

l) Zur Herstellung von Verbindungen der allgemeinen Formel I, in der $R_2$ einen der eingangs erwähnten Oxobuttersäureesterreste darstellt:
Umsetzung einer Verbindung der allgemeinen Formel

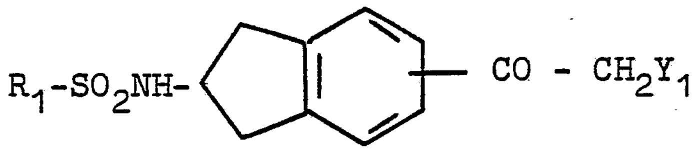

(XIII)

in der

$R_1$ wie eingangs definiert ist und

$Y_1$ eine nukleophile Austrittsgruppe wie ein Halogenatom, z.B. ein Chlor-, Brom-oder Jodatom, oder $Y_1$ zusammen mit dem benachbarten Wasserstoffatom ein Sauerstoffatom darstellt, mit einer Verbindung der allgemeinen Formel

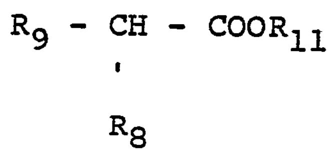

, (XIV)

in der

$R_8$ ein Wasserstoffatom oder eine Alkylgruppe mit 1 bis 3 Kohlenstoffatomen,

$R_9$ ein Wasserstoffatom, eine Alkylgruppe mit 1 bis 3 Kohlenstoffatomen oder eine Alkoxycarbonylgruppe mit insgesamt 2 bis 4 Kohlenstoffatomen, wobei jedoch nur einer der Reste $R_8$ oder $R_9$ eine Alkylgruppe mit 1 bis 3 Kohlenstoffatomen darstellen kann, oder auch, falls $R_9$ eine Alkoxycarbonylgruppe mit insgesamt 2 bis 4 Kohlenstoffatomen darstellt, $R_8$ zusammen mit der CH-Gruppe eine Methylengruppe und $R_{11}$ eine Alkylgruppe mit 1 bis 3 Kohlenstoffatomen darstellen, in Gegenwart einer Base.

Die Umsetzung wird vorzugsweise in einem Lösungsmittel wie Aceton, Dimethylformamid oder Dimethylsulfoxid in Gegenwart einer Base wie Natriumhydroxid, Kaliumhydroxid, Natriumhydrid, Kalium-tert.butylat oder Piperidin/Pyridin, wobei Piperidin/Pyridin auch als Lösungsmittel verwendet werden kann, bei Temperaturen zwischen 0 und 100°C, vorzugsweise bei Temperaturen zwischen 20 und 50°C, durchgeführt.

Erhält man erfindungsgemäß eine Verbindung der allgemeinen Formel I, in der $R_2$ eine Hydroxycarbonylgruppe darstellt oder enthält, so kann diese mittels Veresterung in eine entsprechende Alkoxycarbonylverbindung übergeführt werden.

Die nachträgliche Veresterung wird zweckmäßigerweise in einem Lösungsmittel, z.B. in einem Überschuß des eingesetzten Alkohols wie Methanol, Äthanol oder Isopropanol, in Gegenwart eines die Säure aktivierenden Mittels wie Thionylchlorid oder Chlorwasserstoffgas bei Temperaturen zwischen 0 und 180°C, vorzugsweise jedoch bei der Siedetemperatur des Reaktionsgemisches, durchgeführt.

Die erhaltenen Verbindungen der allgemeinen Formel I können ferner in ihre Enantiomeren aufgetrennt werden. So lassen sich die erhaltenen Verbindungen der allgemeinen Formel I, welche nur ein optisch aktives Zentrum enthalten, nach an sich bekannten Methoden (siehe Alinger N. L. und Elich W. L. in "Topics in Stereochemistry", Vol. 6, Wiley Interscience, 1971) in ihre optischen Antipoden auftrennen, z.B. durch Umkristallisieren aus einem optisch aktiven Lösungsmittel oder durch Umsetzen mit einer, mit der racemischen Verbindung Salze bildenden optisch aktiven Substanz, insbesondere Basen, und Trennen des auf diese Weise erhaltenen Salzgemisches, z.B. auf Grund von verschiedenen Löslichkeiten, in die diastereomeren Salze, aus denen die freien Antipoden durch Einwirkung geeigneter Mittel freigesetzt werden können. Besonders gebräuchliche, optisch aktive Basen sind z.B. die D-und L-Formen von α-Phenyl-äthylamin oder Cinchonidin.

Desweiteren lassen sich die erhaltenen Verbindungen der allgemeinen Formel I mit mindestens 2 asymmetrischen Kohlenstoffatomen auf Grund ihrer physikalischen-chemischen Unterschiede nach an sich bekannten Methoden, z.B. durch Chromatographie und/oder fraktionierte Kristallisation, in ihre Diastereomeren auftrennen. Ein so erhaltenes Enantiomerenpaar läßt sich anschließend in seine optischen Antipoden, wie oben beschrieben, auftrennen. Enthält beispielsweise eine Verbindung der allgemeinen Formel I zwei optisch aktive Kohlenstoffatome, so erhält man die entsprechenden (R R', S S')-und (R S', S R')-Formen.

Desweiteren lassen sich die so erhaltenen neuen Verbindungen der allgemeinen Formel I, falls diese eine Carboxygruppe enthalten, gewünschtenfalls anschließend in ihre Additionssalze mit anorganischen oder organischen Basen, insbesondere für die pharmazeutische Anwendung in ihre physiologisch verträglichen Additionssalze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis XIV erhält man nach literaturbekannten Verfahren bzw. sind literaturbekannt.

Eine als Ausgangsstoff verwendete Verbindung der allgemeinen Formel II erhält man aus einer entsprechenden N-Acylaminoindanyl-verbindung durch Acylierung nach Friedel-Craft, anschließende Entacylierung und gegebenenfalls anschließende Reduktion, Hydrolyse und/oder Veresterung.

Die als Ausgangsstoffe verwendeten Verbindungen der allgemeinen Formeln IV, V, VII, VIII, IX, X und XI erhält man durch Umsetzung einer entsprechenden Aminoverbindung mit einem entsprechenden Sulfonylhalogenid.

Eine als Ausgangsstoffe verwendete Verbindung der allgemeinen Formel XII erhält man durch Umsetzung einer entsprechenden Oxoverbindung mit Hydrazin.

Die eine als Ausgangsstoffe verwendete Verbindung der allgemeinen Formel XIII erhält man durch Halogenierung einer entsprechenden Acetylverbindung.

Wie bereits eingangs erwähnt, weisen die neuen Verbindungen und deren physiologisch verträgliche Additionssalze mit anorganischen oder organischen Basen wertvolle pharmakologische Eigenschaften auf, insbesondere antithrombotische Wirkungen und eine Hemmwirkung auf die Plättchenaggregation. Außerdem stellen sie auch Thromboxanantagonisten dar. Ferner weisen die neuen Pyridazinone der allgemeinen Formel I auf Grund ihrer Hemmwirkung auf die Phosphodiesterase eine Hemmwirkung auf die Tumormetastasierung auf.

Beispielsweise werden die neuen Verbindungen

A = 4-[2-p-Chlorbenzolsulfonamido-indanyl-(5)]-4-oxo-buttersäure,
B = 4-[2-p-Toluolsulfonamido-indanyl-(5)]-4-oxo-buttersäure,
C = [2-p-Chlorbenzolsulfonamido-indanyl-(5)]-essigsäure,
D = 4-[2-p-Chlorbenzolsulfonamido-indanyl-(5)]-buttersäure,
E = 6-[2-p-Toluolsulfonamido-indanyl-(5)]-4,5-dihydropyridazin-3(2H)-on,
F = 6-[2-o-Methoxybenzolsulfonamido-indanyl-(5)]-4,5-dihydropyridazin-3(2H)-on und
G = 5-Methyl-6-[2-benzolsulfonamido-indanyl-(5)]-4,5-dihydropyridazin-3(2H)-on

auf ihre biologischen Eigenschaften wie folgt geprüft:

1. Antithrombotische Wirkung

Methodik

Die Thrombozytenaggregation wird nach der Methode von BORN und CROSS (J. Physiol. 170, 397 (1964)) in plättchenreichem Plasma gesunder Versuchspersonen gemessen. Zur Gerinnungshemmung wird das Blut mit Natriumcitrat 3,14 % im Volumenverhältnis 1:10 versetzt.

Collagen-induzierte Aggregation

Der Verlauf der Abnahme der optischen Dichte der Plättchensuspension wird nach Zugabe der aggregationsauslösenden Substanz photometrisch gemessen und registriert. Aus dem Neigungswinkel der Dichtekurve wird auf die Aggregationsgeschwindigkeit geschlossen. Der Punkt der Kurve, bei dem die größte Lichtdurchlässigkeit vorliegt, dient zur Berechnung der "optical density".

Die Collagen-Menge wird möglichst gering gewählt, aber doch so, daß sich eine irreversibel verlaufende Reaktionskurve ergibt. Verwendet wird das handelsübliche Collagen der Firma Hormonchemie, München.

Vor der Collagen-Zugabe wird das Plasma jeweils 10 Minuten mit der Substanz bei 37°C inkubiert.

Aus den erhaltenen Meßzahlen wird graphisch eine $EC_{50}$ berechnet, die sich auf eine 50%ige Änderung der "optical density" im Sinne einer Aggregationshemmung bezieht.

Die nachfolgende Tabelle enthält die gefundenen Ergebnisse:

| Substanz | $EC_{50}$ [µMol/l] |
|---|---|
| A | 0.37 |
| B | 0.5 |
| C | 0.27 |
| D | 0.8 |
| E | 2.85 |
| F | 0.35 |
| G | 0.25 |

2. Akute Toxizität

Die akute Toxizität der zu untersuchenden Substanzen wurde orientierend an Gruppen von je 10 Mäusen nach oraler Gabe einer Einzeldosis bestimmt (Beobachtungszeit: 14 Tage):

| Substanz | Orientierende akute Toxizität |
|---|---|
| A | 1.000 mg/kg (0 von 10 Tieren gestorben) |
| B | 1.000 mg/kg (0 von 10 Tieren gestorben) |
| C | 1.000 mg/kg (0 von 10 Tieren gestorben) |
| D | 1.000 mg/kg (0 von 10 Tieren gestorben) |
| E | 500 mg/kg (0 von 10 Tieren gestorben) |
| F | 500 mg/kg (0 von 10 Tieren gestorben) |
| G | 500 mg/kg (0 von 10 Tieren gestorben) |

Aufgrund ihrer pharmakologischen Eigenschaften eignen sich die neuen Verbindungen und deren physiologisch verträgliche Additionssalze zur Behandlung und zur Prophylaxe thrombo-embolischer Erkrankungen wie Coronarinfarkt, Cerebralinfarkt, sogen. transient ischaemic attacks, Amaurosis fugax, zur Prophylaxe der Arteriosklerose und zur Metastasenprophylaxe.

Die zur Erzielung einer entsprechenden Wirkung erforderliche Dosierung beträgt zweckmäßigerweise zwei-bis viermal täglich 0,3 bis 4 mg/kg Körpergewicht, vorzugsweise 0,3 bis 2 mg/kg Körpergewicht. Hierzu lassen sich die erfindungsgemäß hergestellten Verbindungen der allgemeinen Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltige Substanzen wie Hartfett oder deren geeignete Gemische, in übliche galenische Zubereitungen wie Tabletten, Dragées, Kapseln, Pulver, Suspensionen oder Zäpfchen einarbeiten.

Die nachfolgenden Beispiele sollen die Erfindung näher erläutern:

Beispiel A

4-[2-Amino-indanyl-(5)]-4-oxo-buttersäure-methylester

1. 4-[2-Acetamido-indanyl-(5)]-4-oxo-buttersäure

Man suspendiert in 500 ml Äthylenchlorid 137,3 g (1,03 Mol) wasserfreies Aluminiumchlorid und 34,2 g (0,34 Mol) Bernsteinsäureanhydrid und trägt unter kräftigem Rühren bei Raumtemperatur 54,5 g (0,311 Mol) 2-Acetamido-indan ein. Man rührt noch zwei Stunden bei Raumtemperatur, versetzt mit Eis und 150 ml konzentrierter Salzsäure und destilliert das Äthylenchlorid mit Wasserdampf ab. Aus der wässrigen Lösung kristallisiert beim Abkühlen 4-(2-Acetamido-indanyl-(5)]-4-oxo-buttersäure aus. Zur Reinigung löst man die Säure in 2n Natronlauge, filtriert und fällt sie mit Salzsäure wieder aus.

Ausbeute: 74,5 g (87 % der Theorie),

Schmelzpunkt: 173-177°C

IR-Spektrum (in KBr): NH bei 3380 $cm^{-1}$, CO bei 1725 + 1680 $cm^{-1}$, Amid-II bei 1530 $cm^{-1}$, CO bei 1640 $cm^{-1}$

2. 4-(2-Amino-indanyl-(5)]-4-oxo-buttersäure-methylester-hydrochlorid

74,5 g 4-[2-Acetylamino-indanyl-(5)]-4-oxo-buttersäure werden 6 Stunden lang mit halbkonzentrierter Salzsäure am Rückfluß gekocht. Man dampft im Vakuum ein und trocknet den Rückstand durch Kochen mit Toluol am Wasserabscheider. Das verbleibende 4-[2-Amino-indanyl-(5)]-4-oxo-buttersäure-hydrochlorid wird zur Veresterung in 600 ml Methanol suspendiert. Man leitet unter Rühren Chlorwasserstoff-Gas bis zur Sättigung ein. Anschließend kühlt man und saugt das ausgefallene Reaktionsprodukt ab.

Ausbeute: 58,6 g (76,5 % der Theorie),

Schmelzpunkt: 198-200°C

$C_{14}H_{18}ClNO_3$ (283,76)

Ber.: C 59,26 H 6,39 N 4,94 Cl 12,49

Gef.: 59,20 6,59 4,95 12,63

Beispiel B

6-[2-Amino-indanyl-(5)]-4,5-dihydro-pyridazin-3(2H)-on

10,0 g (0,035 Mol) 4-[2-Amino-indanyl-(5)]-4-oxo-buttersäure-methylester-hydrochlorid werden in Eisessig mit 8,5 g (0,17 Mol) 99%igem Hydrazin-Hydrat versetzt und 30 Minuten gekocht. Man dampft im Vakuum ein, löst den Rückstand in Wasser und fällt das Reaktionsprodukt durch Neutralisation mit Natriumhydroxid aus.

Ausbeute: 6,5 g (80 % der Theorie),

Schmelzpunkt: 204-205°C (Zers.)

$C_{13}H_{15}N_3O$ (229,28)

Ber.: C 68,10 H 6,59 N 18,33

Gef.: 68,14 6,41 18,35

Beispiel C

[2-Benzolsulfonamido-indanyl-(5)]-methyl-keton

Man suspendiert 29,6 g (0,22 Mol) wasserfreies Aluminiumchlorid in 250 ml Äthylenchlorid und versetzt nacheinander mit 10,5 g (0,133 Mol) Acetylchlorid und mit 24,2 g (0,0885 Mol) 2-(Benzolsulfonamido)-indan. Nach ca. 3 stündigem Rühren bei Raumtemperatur zersetzt man mit Eis und konzentrierter Salzsäure. Das Reaktionsprodukt erhält man beim Einengen der organischen Phase und anschließender Kristallisation aus Cyclohexan/Essigester.

Ausbeute: 21,8 g (78 % der Theorie),
Schmelzpunkt: 119-121 °C
NMR-Spektrum (in $CDCl_3$ -$CD_3OD$):
CH: Multiplett bei 4.1 ppm, 4 aliphat. H: Multiplett bei 2.6 bis 3.4 ppm, $CH_3CO$ bei 2.55 ppm

Beispiel D

3-Methyl-4-[2-amino-indanyl-(5)]-4-oxo-buttersäure-methylester-hydrochlorid

1. 2-Acetamido-5-(2-chlorpropionyl)-indan

Eine Suspension von 40 g (0,3 Mol) wasserfreiem Aluminiumchlorid wird mit 19,0 g (0,147 Mol) 2-Chlorpropionsäurechlorid und anschließend mit 25 g (0,14 Mol) 2-Acetylamino-indan versetzt. Nach 3 Stunden Rühren wird mit Eis und konzentrierter Salzsäure zersetzt. Das Reaktionsprodukt erhält man beim Einengen der organischen Phase.
Ausbeute: 37 g (100 %)
Öl, RF-Wert: 0,2 (Kieselgel-Polygram-Platte; Cyclohexan/Essigester = 1:1)

2. 3-Methyl-4-[2-acetamido-indanyl-(5)]-4-oxo-buttersäure

Man gibt zu einer Lösung von 25 g (0,154 Mol) Malonsäurediäthylester in 40 ml Dimethylsulfoxid unter Rühren 17,8 g (0,154 Mol) Kalium-tert.butanolat und, nachdem eine klare Lösung entstanden war, 38,7 g (0,14 Mol) 2-Acetylamino-5-(2-chlorpropionyl)-indan, gelöst in 30 ml Dimethylsulfoxid. Man rührt 18 Stunden bei Raumtemperatur, versetzt mit Wasser und extrahiert den entstandenen 2-Carbäthoxy-3-methyl-4-oxo-4-[2-acetamido-indanyl-(5)]buttersäureäthylester mit Essigester. Das Reaktionsprodukt wird nach dem Eindampfen als Öl erhalten. Dieses Öl wird ohne weitere Reinigung mit 160 ml 4n Natronlauge versetzt und bei Raumtemperatur 22 Stunden lang gerührt. Nach etwa 5 Stunden entsteht eine klare Lösung. Man wäscht mit Methylenchlorid und fällt die 2-Carboxy-3-methyl-4-oxo-4-[2-acetamido-indanyl-(5)]buttersäure mit Salzsäure aus. Man extrahiert sie mit Essigester und erhält sie nach Eindampfen als Öl.
Ausbeute' 46,6 g (99,8 %).:
RF-Wert: 0,1 (Kieselgel-Polygram-Platten; Essigester als Laufmittel).
Dieses Öl wird zur Decarboxylierung mit 40 ml Diäthylenglykoldimethyläther vermischt und 1,5 Stunden lang auf 120-140°C erhitzt, wobei eine kräftige $CO_2$-Entwicklung einsetzt. Man verdünnt mit Wasser, stellt mit Natronlauge alkalisch, wäscht mit Methylenchlorid und fällt die 3-Methyl-4-[2-acetamido-indanyl-(5)]-4-oxo-buttersäure als langsam erstarrendes Öl aus.
Ausbeute: 31 g (77 % der Theorie).

3. 3-Methyl-4-[2-amino-indanyl-(5)]-4-oxo-buttersäure-methylester-hydrochlorid

Zur Hydrolyse der Acetylamino-Gruppe werden 31 g der vorstehenden Verbindung 8 Stunden lang mit 120 ml halbkonzentrierter Salzsäure gekocht. Man dampft im Vakuum ein und trocknet das verbleibende 3-Methyl-4-oxo-4-[2-amino-indanyl-(5)]-buttersäure-hydrochlorid. Dieses löst man in 400 ml Methanol und leitet anschließend Chlorwasserstoff-Gas bis zur Sättigung ein und läßt über Nacht stehen. Man dampft im Vakuum ein und chromatographiert den Rückstand an 250 g Kieselgel mit Chloroform/Methanol (96:4) als Laufmittel. Man erhält kristallines 3-Methyl-4-[2-amino-indanyl-(5)]-4-oxo-buttersäure-methylester-hydrochlorid.
Ausbeute: 15,5 g (43 % der Theorie),
Schmelzpunkt: 169-173°C
$C_{15}H_{19}NO_3$ x HCl (297,78)
Ber.: C 60,50 H 6,77 N 4,70 Cl 11,91
Gef.: 60,70 6,55 4,81 12,07

Beispiel E

2-Benzolsulfonamido-indan

20 g (0,118 Mol) 2-Amino-indan-hydrochlorid in 100 ml Dioxan werden mit 180 ml halbgesättigter Kaliumcarbonat-Lösung unterschichtet und dann unter kräftigem Rühren tropfenweise mit 26,0 g (0,142 Mol)Benzolsulfonsäurechlorid versetzt. Man rührt zwei Stunden nach, saugt vom Salzniederschlag ab und isoliert das Reaktionsprodukt aus der Dioxan-Phase. Man kristallisiert es aus 150 ml Cyclohexan und 30 ml Essigester um.
Ausbeute: 27,5 g (85,2 % der Theorie),
Schmelzpunkt: 101-102°C
Analog werden erhalten:

2-(p-Chlorbenzolsulfonamido)-indan

Ausbeute: 71 % der Theorie,
Schmelzpunkt: 141-142°C
IR-Spektrum (in Methylenchlorid): NH bei 3360 $cm^{-1}$, $SO_2$ bei 1160 + 1340 $cm^{-1}$

2-(o-Toluolsulfonamido)-indan

Ausbeute: 86 % der Theorie,
Schmelzpunkt: 72-74°C
IR-Spektrum (in Methylenchlorid): NH bei 3360 $cm^{-1}$, $SO_2$ bei 1155 + 1330 $cm^{-1}$

Beispiel F

4-[2-Amino-indanyl-(5)]-buttersäure-methylester

15 g (52,8 mMol) 4-[2-Amino-indanyl-(5)]-4-oxo-buttersäure-methylester werden in 200 ml Eisessig mit 3 ml Perchlorsäure und 3 g 10%igem Palladium auf Kohle versetzt und bei 50°C und 5 bar Wasserstoffdruck hydriert. Nach Aufnahme der berechneten Menge Wasserstoff filtriert man vom Katalysator ab, dampft ein, versetzt den Rückstand mit Natriumhydroxid und extrahiert das Reaktionsprodukt mit Äther. Nach dem Trocknen und Eindampfen erhält man das gewünschte Produkt als Öl.
Ausbeute: 9 g (73 % der Theorie),
RF-Wert: 0,5 (Kieselgel-Polygram-Platten der Fa. Merck; Toluol/Dioxan/Methanol/Ammoniak = 20:50:20:10).
IR-Spektrum (in Methylenchlorid): $OCH_3$ bei 2840 $cm^{-1}$, CO bei 1735 $cm^{-1}$.
NMR-Spektrum (in $CDCl_3$, $CD_3OD$):
CH: Multiplett bei 3.7 ppm, 10 aliphat. H: Multiplett bei 1.7 bis 3.4 ppm, $OCH_3$ bei 3.6 ppm.

Beispiel G

[2-Acetamido-indanyl-(5)]-methyl-keton

Zu einer Suspension von 68 g (0,51 Mol) wasserfreiem Aluminiumchlorid in 350 ml Äthylenchlorid gibt man unter Rühren 24,4 g (0,31 Mol) Acetylchlorid und anschließend 35,5 g (0,20 Mol) 2-Acetamido-indan. Die Reaktionstemperatur steigt dabei auf ca. 50°C an. Man rührt noch 3 Stunden nach, zersetzt mit Eis und konz. Salzsäure, trennt die organische Phase ab und dampft sie ein.
Ausbeute: 42,0 g (96 % der Theorie).
Eine Probe wird aus Isopropanol umkristallisiert.
Schmelzpunkt: 143-145°C
$C_{13}H_{15}NO_2$ (217,27)
Ber.: C 71,87 H 6,96 N 6,45
Gef.: 71,81 6,67 6,56

Beispiel H

[2-Amino-indanyl-(5)]-essigsäure-methylester

23,6 g (0,108 Mol) [2-Acetamido-indanyl-(5)]-methyl-keton, 5,45 g (0,17 Mol) Schwefel und 22 g (0,25 Mol) Morpholin werden 6,5 Stunden lang zum Sieden erhitzt. Nach dem Abkühlen versetzt man mit Essigester. Das [2-Acetamido-indanyl-(5)]-essigsäure-thiomorpholid fällt kristallin an. Man reinigt es durch Kristallisation aus Isopropanol.
Ausbeute: 29,7 g (86 % der Theorie).
Schmelzpunkt: 171-174°C.
Dieses Thiomorpholid kocht man mit 37 g (0,56 Mol) Kaliumhydroxid in 50 ml Wasser und 50 ml Äthanol 16 Stunden lang. Man dampft ein, versetzt den Rückstand mit überschüssiger Salzsäure und dampft erneut zur Trockene ein. Man digeriert den Rückstand mit Methanol, saugt vom Kaliumchlorid ab und leitet in das Filtrat Chlorwasserstoff-Gas bis zur Sättigung ein. Nach Stehen über Nacht wird eingedampft. Das Hydrochlorid des [2-Amino-indanyl-(5)]-essigsäure-methylester wird mit Aceton und Äther verrührt und abgesaugt.
Ausbeute: 13,9 g (62 % der Theorie),
Schmelzpunkt: 120-125°C,
$C_{12}H_{16}ClNO_2$ (241,72)
Ber.: Cl 14,67 N 5,79
Gef.: 14,65 5,87

Beispiel I

2-[2-Amino-indanyl-(5)]-propionsäure-methylester-hydrochlorid

a) 1-[2-Acetamido-indanyl-(5)]-1-äthanol

24 g (0,11 mMol) [2-Acetamido-indanyl-(5)]-methylketon (Beispiel G) in 200 ml Methanol werden bei Raumtemperatur mit einer Lösung von 4,6 g (0,12 Mol) Natriumborhydrid in 25 ml Wasser versetzt. Nach 2,5 Stunden dampft man im Vakuum ein, verdünnt mit Eiswasser und säuert an. Man extrahiert das Reaktionsprodukt mit Methylenchlorid/Methanol = 9:1, trocknet die organische Phase und entfernt im Vakuum das Lösungsmittel.
Ausbeute: 18,6 g (77 % der Theorie),
Schmelzpunkt: 127-129°C
IR-Spektrum (in Methylenchlorid): OH bei 3610 $cm^{-1}$,
CONH bei 1670 und 1510 $cm^{-1}$

b) 1-[2-Acetamido-indanyl-(5)]-1-chloräthan

25 g (0,11 Mol) 1-[2-Acetamido-indanyl-(5)]-1-äthanol werden in 280 ml Methylenchlorid suspendiert und langsam mit 15 g (0,12 Mol) Thionylchlorid versetzt. Es bildet sich eine klare Lösung. Diese wird nach einer Stunde durch Waschen mit einer gesättigten Lösung von Natriumhydrogencarbonat entsäuert. Nach dem Trocknen und Eindampfen erhält man das gewünschte Reaktionsprodukt.
Ausbeute: 26,1 g (97 % der Theorie),
Schmelzpunkt: 102-104°C
$C_{13}H_{16}ClNO$ (237,73)
Ber.: C 65,68 H 6,78 N 5,89 Cl 14,91
Gef.: 65,72 6,48 6,23 14,68
IR-Spektrum (in Methylenchlorid): NH bei 3440 $cm^{-1}$,
CONH bei 1670 und 1510 $cm^{-1}$

c) 2-[2-Acetamido-indanyl-(5)]-1-propionsäurenitril

13,3 g (0,056 Mol) 1-[2-Acetamido-indanyl-(5)]-1-chlor-äthan werden mit 3,3 g (0,067 Mol) Natriumcyanid in 45 ml Dimethylsulfoxid 7 Stunden lang auf 70°C erhitzt. Man versetzt mit 200 ml Wasser und extrahiert mit Essigester. Die organische Phase wird getrocknet und im Vakuum eingeengt.
Ausbeute: 9,3 g (73 % der Theorie),
Öl, Rf-Wert: 0,5 (Kieselgel-Polygram-Platten SIL G/UV der Fa. Macherey-Nagel, Düren; Laufmittel: Äthylenchlorid/Isopropanol = 9:1).

d) 2-[2-Amino-indanyl-(5)]-propionsäure-methylester-hydrochlorid

Eine Lösung von 12,5 g (0,05 Mol) 2-[2-Acetamido-indanyl-(5)]-propionsäurenitril in 150 ml Methanol wird mit Chlorwasserstoff-Gas gesättigt. Man kocht anschließend 40 Stunden lang. Die Lösung wird im Vakuum eingeengt. Es verbleibt ein kristallines Material.
Ausbeute: 10,0 g (79 % der Theorie),
IR-Spektrum (in KBr): Ester-CO bei 1730 $cm^{-1}$

Beispiel K

[2-p-Toluolsulfonamido-indanyl-(5)]-chlormethyl-keton

a) 2-p-Toluolsulfonamido-indan

Hergestellt analog Beispiel E aus 2-Amino-indan-hydrochlorid und p-Toluolsulfochlorid.
Ausbeute: 90 % der Theorie,
Schmelzpunkt: 130-132°C (Essigester/Cyclohexan).

b) [2-p-Toluolsulfonamido-indanyl-(5)]-chlormethyl-keton

25,4 g (0,19 Mol) wasserfreies Aluminiumchlorid werden in 80 ml Äthylenchlorid suspendiert und mit 11,1 g (0,095 Mol) Chloracetylchlorid versetzt. Anschließend gibt man 22,8 g (0,079 Mol) 2-p-Toluolsulfonamido-indan zu und rührt 2 Stunden bei Raumtemperatur. Anschließend zersetzt man mit Eis und Salzsäure, isoliert das Reaktionsprodukt aus der organischen Phase und kristallisiert es aus Essigester Cyclohexan um.
Ausbeute: 23,5 g (31,4 % der Theorie),
Schmelzpunkt: 132-134°C
$C_{18}H_{18}ClNO_3S$ (363,86)
Ber.: C 59,42 H 4,99 N 3,85 Cl 9,74 S 8,81
Gef.: 59,22 4,86 3,58 10,00 9,03
Analog wird dargestellt:
[2-p-Chlorbenzolsulfonamido-indanyl-(5)]-chlormethylketon
Ausbeute: 91 % der Theorie,
Schmelzpunkt: 167-170°C (aus Äthylenchlorid)

Beispiel 1

4-[2-Benzolsulfonamido-indanyl-(5)]-4-oxo-buttersäure-methylester

6,4 g (22,5 mMol) 4-[2-Amino-indanyl-(5)]-4-oxo-buttersäure-methylester-hydrochlorid werden in 35 ml Pyridin suspendiert, mit 5,0 g (25 mMol) Benzolsulfonsäurechlorid und anschließend mit 2,7 g (27 mMol) Triäthylamin versetzt. Man rührt 3 Stunden, dampft im Vakuum ein, versetzt mit Salzsäure und schüttelt das Reaktionsprodukt mit Essigester aus. Man reinigt es durch Chromatographie an 350 g Kieselgel mit Cyclohexan/Essigester = 1:1.
Ausbeute: 8,6 g (99 % der Theorie)).

Öl, RF-Wert: 0,4 (Kieselgel-Polygram-Platten; Cyclohexan/Essigester = 1:1)
IR-Spektrum (in Methylenchlorid): NH bei 3360 $cm^{-1}$, Ester-CO bei 1735 $cm^{-1}$, Keton-CO bei 1680 $cm^{-1}$, $SO_2$ bei 1155 + 1340 $cm^{-1}$
NMR-Spektrum (in $CDCl_3$ -$CD_3OD$):
CH und 8 aliphat. H als Multipletts bei 4.1 bzw. 2.6 bis 3.4 ppm, $OCH_3$ bei 3.7 ppm.

Beispiel 2

4-[2-Benzolsulfonamido-indanyl-(5)]-4-oxo-buttersäure

8,3 g (21,4 mMol) 4-[2-Benzolsulfonamido-indanyl-(5)]-4-oxo-buttersäure-methylester in 20 ml Äthanol werden mit 2,7 ml 15n Natronlauge versetzt und 2 Stunden gekocht. Man dampft im Vakuum ein, nimmt den Rückstand in Wasser auf und fällt das Reaktionsprodukt mit Salzsäure aus. Man kristallisiert es aus Essigester/Diisopropyläther.
Ausbeute: 6,4 g (80 % der Theorie),
Schmelzpunkt: 134-136°C
$C_{19}H_{19}NO_5S$ (373,43)
Ber.: C 61,11 H 5,13 N 3,75 S 8,59
Gef.: 61,20 5,37 3,76 8,81

Beispiel 3

4-[2-p-Fluorbenzolsulfonamido-indanyl-(5)]-4-oxo-buttersäure-methylester

Hergestellt analog Beispiel 1 aus 4-[2-Amino-indanyl-(5)]-4-oxo-buttersäure-methylester-hydrochlorid und p-Fluorbenzolsulfonsäurechlorid in Pyridin und Triäthylamin.
Ausbeute: 80 % der Theorie,
Schmelzpunkt: 130-132°C (Cyclohexan/Essigester = 1:1)
$C_{20}H_{20}FNO_5S$ (405,45)
Ber.: C 59,25 H 4,97 N 3,45 S 7,91
Gef.: 59,01 5,10 3,39 8,16

Beispiel 4

4-[2-p-Fluorbenzolsulfonamido-indanyl-(5)]-4-oxo-buttersäure

Hergestellt analog Beispiel 2 aus 4-[2-p-Fluorbenzolsulfonamido-indanyl-(5)]-4-oxo-buttersäure-methylester durch Hydrolyse mit Natriumhydroxid.
Ausbeute: 75 % der Theorie,
Die Substanz kristallisiert aus Isopropanol in zwei Modifikationen mit den Schmelzpunkten 133-135°C und 174-176°C.
$C_{19}H_{18}FNO_5S$ (391,42)
Ber.: C 58,30 H 4,64 N 3,58 S 8,19
Substanz mit Schmelzpunkt 133-135°C
Gef.: C 58,02 H 4,60 N 3,56 S 8,21
Substanz mit Schmelzpunkt 174-176°C
Gef.: C 58,33 H 4,68 N 3,63 S 8,41

Beispiel 5

4-[2-p-Toluolsulfonamido-indanyl-(5)]-4-oxo-buttersäure-methyl-ester

6,0 g (21,1 mMol) 4-[2-Amino-indanyl-(5)]-4-oxo-buttersäure-methylester-hydrochlorid werden in 60 ml Chloroform suspendiert und mit 5,0 g (25,3 mMol) p-Toluolsulfonsäurechlorid versetzt. Unter kräftigem Rühren tropft man 5,1 g (50 mMol) Triäthylamin zu, wobei eine klare Lösung entsteht. Nach 20 Stunden bei Raumtemperatur wäscht man mit Wasser, dampft die Chloroform-Lösung ein und kristallisiert den Rückstand aus Methanol um.
Ausbeute: 7,1 g (84 % der Theorie),
Schmelzpunkt: 108-110°C
$C_{21}H_{23}NO_5S$ (401,48)
Ber.: C 62,83 H 5,77 N 3,49 S 7,99
Gef.: 62,70 5,70 3,45 7,80

Beispiel 6

4-[2-p-Toluolsulfonamido-indanyl-(5)]-4-oxo-buttersäure

Hergestellt analog Beispiel 2 aus 4-[2-p-Toluolsulfonamido-indanyl-(5)]-4-oxo-buttersäure-methylester durch Hydrolyse mit Natriumhydroxid.
Ausbeute: 72 % der Theorie,
Die Substanz kristallisiert aus Essigester in zwei Modifikationen mit den Schmelzpunkten 143-145°C und 161-163°C.
$C_{20}H_{21}NO_5S$ (487.46)
Ber.: C 62,20 H 5,46 N 3,62 S 8,27
Substanz mit Schmelzpunkt 143-145°C.
Gef.: C 62,24 H 5,29 N 3,56 S 8,37
Substanz mit Schmelzpunkt 161-163°C
Gef.: C 62,30 H 5,29 N 3,64 S 8,37

Beispiel 7

4-[2-o-Methoxybenzolsulfonamido-indanyl-(5)]-4-oxo-buttersäure-methylester

Hergestellt analog Beispiel 5 aus 4-[2-Amino-indanyl-(5)]-4-oxo-buttersäure-methylester-hydrochlorid und o-Methoxybenzolsulfonsäurebromid in Chloroform in Gegenwart von Triäthylamin.
Ausbeute: 90 % der Theorie,
Schmelzpunkt: 115-119°C
NMR-Spektrum (in $CDCl_3$ -$CD_3OD$):
CH und 8 aliphat. H als Multipletts bei 4.1 bzw. 2.6 bis 3.4 ppm;
zwei $OCH_3$ bei 3.7 und 3.9 ppm

Beispiel 8

4-[2-o-Methoxybenzolsulfonamido-indanyl-(5)]-4-oxo-buttersäure

Hergestellt analog Beispiel 2 aus 4-[2-o-Methoxybenzolsulfonamido-indanyl-(5)]-4-oxo-buttersäure-methylester durch Hydrolyse mit Natriumhydroxid.
Ausbeute: 59 % der Theorie,
Schmelzpunkt: 148-150°C (Äthylenchlorid/Diisopropyläther) $C_{20}H_{21}NO_6S$ (403,46)
Ber.: C 59,54 H 5,25 N 3,47 S 7,95
Gef.: 59,70 5,24 3,39 8,18

Beispiel 9

4-[2-p-Methoxybenzolsulfonamido-indanyl-(5)]-4-oxo-buttersäure-methylester

Hergestellt analog Beispiel 5 aus 4-[2-Amino-indanyl-(5)]-4-oxo-buttersäure-methylester-hydrochlorid und p-Methoxybenzolsulfonsäurechlorid in Chloroform in Gegenwart von Triäthylamin.
Ausbeute: 86 % der Theorie,
Schmelzpunkt: 101-103°C (Methanol)
$C_{21}H_{23}NO_6S$ (417,48)
Ber.: C 60,42 H 5,55 N 3,36 S 7,68
Gef.: 60,30 5,47 3,52 7,90

Beispiel 10

4-[2-p-Methoxybenzolsulfonamido-indanyl-(5)]-4-oxo-buttersäure

Hergestellt analog Beispiel 2 aus 4-[2-p-Methoxybenzolsulfonamido-indanyl-(5)]-4-oxo-buttersäure-methylester durch Hydrolyse mit Natriumhydroxid.
Ausbeute: 90 % der Theorie,
Schmelzpunkt: 131-133°C (Äthylenchlorid)
$C_{20}H_{21}NO_6S$ (403,46)
Ber.: C 59,54 H 5,25 N 3,47 S 7,95
Gef.: 59,25 5,40 3,38 7,86

Beispiel 11

3-Methyl-4-[2-benzolsulfonamido-indanyl-(5)]-4-oxo-buttersäure-methylester

Hergestellt analog Beispiel 1 aus 3-Methyl-4-[2-amino-indanyl-(5)]-4-oxo-buttersäure-methylester-hydrochlorid und Benzolsulfonsäurechlorid.
Ausbeute: 98 % der Theorie,
Öl, Rf-Wert: 0,8 (Kieselgel-Polygram-Platten; Chloroform/Methanol = 95:5 als Laufmittel)
$C_{21}H_{23}NO_5S$ (401,48)
Ber.: C 62,83 H 5,77 N 3,49 S 7,99
Gef.: 62,52 5,67 3,60 7,98
NMR-Spektrum (in $CDCl_3$-$CD_3OD$):
CH und $CH(CH_3)$: Multiplett bei 4.0 ppm 6 aliphat. H: Multiplett bei 2.2 bis 3.4 ppm
$CH_3$: Dublett bei 1.15 ppm

Beispiel 12

3-Methyl-4-[2-benzolsulfonamido-indanyl-(5)]-4-oxo-buttersäure

Hergestellt analog Beispiel 2 aus 3-Methyl-4-[2-benzolsulfonamido-indanyl-(5)]-4-oxo-buttersäure-methylester durch Hydrolyse mit Natriumhydroxid.
Ausbeute: 36 % der Theorie,
Schmelzpunkt: 101-103°C
$C_{20}H_{21}NO_5S$ (387,46)
Ber.: N 3,62 S 8,27
Gef.: 3,40 8,04

Beispiel 13

3-Methyl-4-[2-p-fluorbenzolsulfonamido-indanyl-(5)]-4-oxo-buttersäure-methylester

Hergestellt analog Beispiel 1 aus 3-Methyl-4-[2-amino-indanyl-(5)]-4-oxo-buttersäure-methylester-hydrochlorid und p-Fluorbenzolsulfonsäurechlorid.
Ausbeute: 98 % der Theorie,
Öl, RF-Wert: 0,7 (Polygram-Kieselgel-Platten; Chloroform/Methanol = 95:5 als Laufmittel)
$C_{19}H_{18}FNO_5S$ (391,42)
Ber.: C 58,30 H 4,64 N 3,58 S 8,19
Gef.: 58,33 4,68 3,63 8,41
NMR-Spektrum (in $CDCl_3$ -$CD_3OD$):
CH und $CH(CH_3)$: Multiplett bei 4.0 ppm 6 aliphat. H: Multiplett bei 2.2 bis 3.4 ppm
$OCH_3$ bei 3.65 ppm

Beispiel 14

3-Methyl-4-[2-p-fluorbenzolsulfonamido-indanyl-(5)]-4-oxo-buttersäure

Hergestellt analog Beispiel 2 aus 3-Methyl-4-[2-p-fluorbenzolsulfonamido-indanyl-(5)]-4-oxo-buttersäure-methylester durch Hydrolyse mit Natriumhydroxid.
Ausbeute: 69 % der Theorie,
Schmelzpunkt: 155-157°C (Toluol/Essigester)
$C_{20}H_{20}FNO_5S$ (405,45)
Ber.: C 59,25 H 4,97 N 3,45 S 7,91
Gef.: 59,00 5,14 3,36 8,17

Beispiel 15

3-Methyl-4-[2-p-toluolsulfonamido-indanyl-(5)]-4-oxo-buttersäure-methylester

Hergestellt analog Beispiel 1 aus 3-Methyl-4-[2-amino-indanyl-(5)]-4-oxo-buttersäure-methylester-hydrochlorid und p-Toluolsulfonsäurechlorid.
Ausbeute: 70 % der Theorie,
Schmelzpunkt: 112-115°C (Diisopropyläther/Essigester = 100:5)
$C_{22}H_{25}NO_5S$ (415,51)
Ber.: C 63,60 H 6,06 N 3,37 S 7,72
Gef.: 63,32 5,97 3,49 7,54

Beispiel 16

3-Methyl-4-[2-p-toluolsulfonamido-indanyl-(5)]-4-oxo-buttersäure

Hergestellt analog Beispiel 2 aus 3-Methyl-4-[2-p-toluolsulfonamido-indanyl-(5)]-4-oxo-buttersäure-methylester durch Hydrolyse mit Natriumhydroxid.
Ausbeute: 73 % der Theorie,
Schmelzpunkt: 137-139°C (aus Toluol/Essigester)
$C_{21}H_{23}NO_5S$ (401,48)
Ber.: C 62,83 H 5,77 N 3,49 S 7,99
Gef.: 63,00 5,68 3,47 8,04

Beispiel 17

4-[2-p-Chlorbenzolsulfonamido-indanyl-(5)]-4-oxo-buttersäure

Man suspendiert 38,5 g (0,289 Mol) wasserfreies Aluminiumchlorid und 12,4 g (0,124 Mol) Bernsteinsäureanhydrid in 250 ml Äthylenchlorid und versetzt anschließend unter kräftigem Rühren mit 25,4 g (0,082 Mol) 2-p-Chlorbenzolsulfonamido-indan. Dabei entsteht eine klare Lösung. Nach 3 Stunden zersetzt man mit Eis und Salzsäure und isoliert das Reaktionsprodukt aus der organischen Phase. Man kristallisiert es aus Eisessig um.
Ausbeute: 13,8 g (41 % der Theorie),
Schmelzpunkt: 148-150°C
$C_{19}H_{18}ClNO_5S$ (407,87)
Ber.: C 55,95 H 4,45 N 3,43 Cl 8,69 S 7,86
Gef.: 55,91 4,37 3,51 8,90 7,79

Beispiel 18

4-[2-o-Toluolsulfonamido-indanyl-(5)]-4-oxo-buttersäure

Hergestellt analog Beispiel 17 aus 2-(o-Toluolsulfonamido)-indan und Bernsteinsäureanhydrid.
Ausbeute: 40 % der Theorie,
Schmelzpunkt: 110-112°C (Cyclohexan/Essigester)
$C_{20}H_{21}NO_5S$ (387,46)
Ber.: C 62,00 H 5,46 N 3,62 S 8,28
Gef.: 62,17 5,61 3,53 8,41

Beispiel 19

4-[2-o-Toluolsulfonamido-indanyl-(5)]-4-oxo-buttersäure-methylester

42,7 g (0,1 Mol) 4-[2-o-Toluolsulfonamido-indanyl-(5)]-4-oxo-buttersäure in 100 ml Methanol werden bei -45°C mit 17,9 g (0,15 Mol) Thionylchlorid versetzt. Man läßt einige Stunden bei Zimmertemperatur stehen, dampft im Vakuum ein und chromatographiert den Rückstand mit Cyclohexan/Essigester = 1:1 an Kieselgel. Die Fraktionen mit einem RF-Wert von 0,6 (Kieselgel-Polygram-Platten; Cyclohexan/Essigester = 1:1) werden vereinigt und eingedampft. Man erhält das Reaktionsprodukt als ein Öl.
Ausbeute: 23 g (57 % der Theorie),
$C_{21}H_{23}NO_5S$ (401,48)
Ber.: N 3,49 S 7,99
Gef.: 3,31 7,93
IR-Spektrum (in Methylenchlorid):
NH bei 3370 $cm^{-1}$, Ester-CO bei 1735 $cm^{-1}$, Keton-CO bei 1675 $cm^{-1}$, $SO_2$ bei 1160 + 1330 $cm^{-1}$

Beispiel 20

4-[2-Benzolsulfonamido-indanyl-(5)]-4-oxo-buttersäure

Hergestellt analog Beispiel 17 aus 2-Benzolsulfonamido-indan und Bernsteinsäureanhydrid.
Ausbeute: 38 % der Theorie,
Schmelzpunkt: 131-134°C (Diisopropyläther)
$C_{19}H_{19}NO_5S$ (373,43)
Ber.: C 61,11 H 5,13 N 3,75 S 8,59
Gef.: 61,20 5,37 3,76 8,81

Beispiel 21

6-[2-p-Fluorbenzolsulfonamido-indanyl-(5)]-4,5-dihydro-pyridazin-3(2H)-on

2,9 g (12,6 mMol) 6-[2-Amino-indanyl-(5)]-4,5-dihydro-pyridazin-3(2H)-on werden in einer Mischung von 30 ml Dioxan und 10 ml Aceton gelöst und mit 1,42 g (14 mMol) Triäthylamin und 2,8 g p-Fluorbenzolsulfonsäurechlorid versetzt. Nach 2 Stunden wird eingedampft, der Rückstand mit Wasser versetzt, das Reaktionsprodukt abgesaugt und aus Eisessig umkristallisiert.
Ausbeute: 72 % der Theorie,
Schmelzpunkt: 209-211 °C
$C_{19}H_{18}FN_3O_3S$ (387,43)
Ber.: C 58,90 H 4,68 N 10,85 S 8,28
Gef.: 58,76 4,71 10,72 8,59

Beispiel 22

6-[2-p-Toluolsulfonamido-indanyl-(5)]-4,5-dihydro-pyridazin-3(2H)-on

Hergestellt analog Beispiel 21 aus 6-[2-Amino-indanyl-(5)]-4,5-dihydro-pyridazin-3(2H)-on und p-Toluolsulfonsäurechlorid.
Ausbeute: 90 % der Theorie,
Schmelzpunkt: 185-187 °C (n-Propanol)
$C_{20}H_{21}N_3O_3S$ (383,47)
Ber.: C 62,64 H 5,52 N 10,96 S 8,36
Gef.: 62,70 5,64 10,92 8,31

Beispiel 23

6-[2-Benzolsulfonamido-indanyl-(5)]-4,5-dihydro-pyridazin-3(2H)-on

3,0 g (8 mMol) 4-[2-Benzolsulfonamido-indanyl-(5)]-4-oxo-buttersäure und 0,53 g (10,4 mMol) 99%iges Hydrazinhydrat werden in 12 ml Eisessig 3 Stunden lang gekocht. Man dampft im Vakuum ein, versetzt den Rückstand mit Wasser, neutralisiert mit Sodalösung, saugt das Reaktionsprodukt ab und kristallisiert es aus n-Propanol um.
Ausbeute: 2,7 g (91 % der Theorie),
Schmelzpunkt: 178-180 °C
$C_{19}H_{19}N_3O_3S$ (369,44)
Ber.: C 61,77 H 5,18 N 11,37 S 8,68
Gef.: 61,74 5,37 11,21 8,95

Beispiel 24

6-[2-p-Chlorbenzolsulfonamido-indanyl-(5)]-4,5-dihydro-pyridazin-3(2H)-on

Hergestellt analog Beispiel 23 aus 4-[2-p-Chlorbenzolsulfonamido-indanyl-(5)]-4-oxo-buttersäure und Hydrazinhydrat in Eisessig.
Ausbeute: 38 % der Theorie,
Schmelzpunkt: 204-206 °C (n-Propanol)
$C_{19}H_{18}ClN_3O_3S$ (403,89)
Ber.: C 56,50 H 4,49 N 10,40 Cl 8,78 S 7,94
Gef.: 56,50 4,56 10,29 8,64 7,82

Beispiel 25

6-[2-o-Methoxybenzolsulfonamido indanyl-(5)]-4,5-dihydro-pyridazin-3(2H)-on

Hergestellt analog Beispiel 23 aus 4-[2-o-Methoxybenzolsulfonamido-indanyl-(5)]-4-oxo-buttersäure und Hydrazinhydrat in Eisessig.
Ausbeute: 63 % der Theorie,
Schmelzpunkt: 224-227°C (Äthylenchlorid)
$C_{20}H_{21}N_3O_4S$ (399,47)
Ber.: C 60,14 H 5,30 N 10,52 S 8,03
Gef.: 59,96 5,25 10,74 7,95

Beispiel 26

6-[2-p-Methoxybenzolsulfonamido-indanyl-(5)]-4,5-dihydro-pyridazin-3(2H)-on

5,0 g (12 mMol) 4-[2-p-Methoxybenzolsulfonamido-indanyl-(5)]-4-oxo-buttersäuremethylester und 3,0 g (60 mMol) 99%iges Hydrazinhydrat werden in 25 ml Eisessig 30 Minuten lang gekocht. Man dampft ein, versetzt den Rückstand mit Wasser, saugt das Reaktionsprodukt ab und kristallisiert es aus n-Propanol um.
Ausbeute: 4,3 g (90 % der Theorie),
Schmelzpunkt: 192-193°C
$C_{20}H_{21}N_3O_4S$ (399,47)
Ber.: C 60,14 H 5,30 N 10,52 S 8,03
Gef.: 59,91 5,17 10,82 8,06

Beispiel 27

6-[2-o-Toluolsulfonamido-indanyl-(5)]-4,5-dihydro-pyridazin-3(2H)-on

Hergestellt analog Beispiel 26 aus 4-[2-o-Toluolsulfonamido-indanyl-(5)]-4-oxo-buttersäure-methylester und Hydrazinhydrat in Eisessig.
Ausbeute: 26 % der Theorie,
Schmelzpunkt: 191-193°C (n-Propanol)
$C_{20}H_{21}N_3O_3S$ (383,47)
Ber.: C 62,64 H 5,52 N 10,96 S 8,36
Gef.: 62,95 5,30 10,80 8,43

Beispiel 28

5-Methyl-6-[2-benzolsulfonamido-indanyl-(5)]-4,5-dihydro-pyridazin-3(2H)-on

Hergestellt analog Beispiel 23 aus 3-Methyl-4-[2-benzolsulfonamido-indanyl-(5)]-4-oxobuttersäure und Hydrazinhydrat in Eisessig.
Ausbeute: 80 % der Theorie,
Schmelzpunkt: 196-198°C (n-Propanol)
$C_{20}H_{21}N_3O_3S$ (383,47)
Ber.: C 62,64 H 5,52 N 10,96 S 8,36
Gef.: 62,80 5,49 11,16 8,49

Beispiel 29

5-Methyl-6-[2-p-toluolsulfonamido-indanyl-(5)]-4,5-dihydro-pyridazin-3(2H)-on

Hergestellt analog Beispiel 26 aus 3-Methyl-4-[2-p-toluolsulfonamido-indanyl-(5)]-4-oxo-buttersäure und Hydrazinhydrat in Eisessig.
Ausbeute: 83 % der Theorie,
Schmelzpunkt: 204-207°C (n-Propanol)
$C_{21}H_{23}N_3O_3S$ (397,50)
Ber.: C 63,46 H 5,83 N 10,57 S 8,07
Gef.: 63,80 5,92 10,48 8,01

Beispiel 30

[2-Benzolsulfonamido-indanyl-(5)]-carbonsäure

Zu einer Lösung von 8,5 g (0,214 Mol) Natriumhydroxid in 85 ml Wasser gibt man bei -10°C 12,5 g (0,0795 Mol) Brom und anschließend unter kräftigem Rühren eine Lösung von 5 g (0,015 Mol) [2-Benzolsulfonamido-indanyl-(5)]-methyl-keton in 20 ml Dioxan. Man rührt 2 Stunden bei Raumtemperatur, zerstört das überschüssige Brom durch Zugabe von Natriumhydrogensulfit, wäscht die alkalische Phase mit Äther, säuert mit Salzsäure an und extrahiert das Reaktionsprodukt mit Methylenchlorid. Nach dem Eindampfen verbleiben 5,3 g harzige Substanz, welche aus Eisessig und dann aus Methanol/Wasser umkristallisiert wird.
Ausbeute: 1,42 g (37 % der Theorie),
Schmelzpunkt: 180-182°C
$C_{16}H_{15}NO_4S$ (317,36)
Ber.: C 60,55 H 4,76 N 4,41 S 10,10
Gef : 60,60 4,58 4,38 10,23

Beispiel 31

[2-Benzolsulfonamido-indanyl-(5)]-essigsäure

8,3 g (26 mMol) [2-Benzolsulfonamido-indanyl-(5)]-methyl-keton, 2,1 g (65 mMol) Schwefel und 5,7 g (65 mMol) Morpholin werden 2 Stunden auf 135°C erhitzt. Man versetzt mit Eis und Salzsäure und extrahiert das [2-Benzolsulfonamido-indanyl-(5)]-essigsäure-thiomorpholid mit Essigester. Zur Reinigung chromatographiert man es an Kieselgel mit Äthylenchlorid/Essigester = 8:2 und erhält 10,6 g (97 % der Theorie) als Harz. Anschließend erhitzt man unter Rückfluß 2,5 Stunden mit 10 g Kaliumhydroxid in 100 ml Wasser und 40 ml Äthanol. Man engt im Vakuum ein, wäscht die alkalische Phase mit Methylenchlorid und fällt das Reaktionsprodukt mit Salzsäure aus. Man kristallisiert es aus Isopropanol und aus Essigester/Diisopropyläther um.
Ausbeute: 3,0 g (34 % der Theorie),
Schmelzpunkt: 109-111°C
$C_{17}H_{17}NO_4S$ (331,39)
Ber.: C 61,62 H 5,17 N 4,23 S 9,68
Gef.: 61,65 5,30 4,16 9,55

Beispiel 32

3-[2-Benzolsulfonamido-indanyl-(5)]-crotonsäure-äthylester

Man versetzt eine Lösung von 8,3 g (26,3 mMol) [2-Benzolsulfonamido-indanyl-(5)]-methyl-keton und 7,4 g (33 mMol) Phosphonoessigsäure-triäthylester in 30 ml Dimethylformamid mit 6,5 g (65,5 mMol) Kalium-tert.butanolat und erhitzt 11 Stunden auf 60°C. Man verdünnt mit Wasser, säuert an und extrahiert das Reaktionsprodukt mit Äther. Zur Reinigung chroma tographiert man es mit Cyclohexan/Essigester = 1:1 an Kieselgel.
Ausbeute: 9,1 g (90 % der Theorie),
Öl, RF-Wert: 0,7 (Kieselgel-Polygram-Platten; Cyclohexan/Essigester als Laufmittel).

Beispiel 33

3-[2-Benzolsulfonamido-indanyl-(5)]-crotonsäure

9,1 g (23,6 mMol) 3-[2-Benzolsulfonamido-indanyl-(5)]-crotonsäure-äthylester werden in 100 ml Äthanol mit 3,4 ml 15n Natronlauge versetzt und eine Stunde gekocht. Beim Abkühlen fällt das Natriumsalz aus. Dieses wird abgesaugt und in Wasser gelöst. Durch Zugabe von Salzsäure fällt man die freie Säure aus, welche aus Isopropanol umkristallisiert wird.
Ausbeute: 2,1 g (25 % der Theorie),
Schmelzpunkt: 159-161 °C
$C_{19}H_{19}NO_4S$ (357,43)
Ber.: C 63,85 H 5,36 N 3,92 S 8,97
Gef.: 64,03 5,37 3,76 8,82
NMR-Spektrum (in $CDCl_3$-$CD_3OD$):
1 olefin. H bei 6.15 ppm, $CH_3$ bei 2.55 ppm, CH und 4 aliphat. H: Multiplett bei 4.1 bzw. bei 2.6-3.4 ppm.

Beispiel 34

3-[2-Benzolsulfonamido-indanyl-(5)]-buttersäure-methylester

3,2 g (8,95 mMol) 3-[2-Benzolsulfonamido-indanyl-(5)]-crotonsäure in 50 ml Dioxan werden in Gegenwart von Raney-Nickel bei Raumtemperatur unter 3,5 bar Wasserstoffdruck hydriert. Man filtriert, dampft ein, löst den Rückstand in 20 ml Methanol und tropft bei -50°C 1,5 ml Thionylchlorid zu. Man läßt über Nacht bei Raumtemperatur stehen, dampft erneut ein und chromatographiert den Rückstand mit Äthylenchlorid an Kieselgel. Die Fraktionen mit einem RF-Wert von 0,6 werden vereinigt und eingedampft. Man erhält ein farbloses Öl.
Ausbeute: 2,4 g (72 % der Theorie)
NMR-Spektrum (in $CDCl_3$ -$CD_3OD$):
CH und 7 aliphat. H als Multipletts bei 4.0 bzw. bei 2.4-3.4 ppm,
$CH_3$ = Dublett bei 1.15 ppm.

Beispiel 35

3-[2-Benzolsulfonamido-indanyl-(5)]-buttersäure

2,4 g 3-[2-Benzolsulfonamido-indanyl-(5)]-buttersäure-methylester werden in 10 ml Äthanol gelöst, mit 2 ml 15n Natronlauge versetzt und durch 1-stündiges Kochen verseift. Man dampft ein, löst den Rückstand in Wasser und fällt die freie Säure als schaumiges Material durch Zugabe von Salzsäure aus.
Ausbeute: 2,0 g (87 % der Theorie)
Schmelzpunkt: > 40°C.
$C_{19}H_{21}NO_4S$ (359,45)
Ber.: C 63,49 H 5,89 N 3,90 S 8,92
Gef.: 63,88 6,01 3,83 8,66

Beispiel 36

3-[2-Benzolsulfonamido-indanyl-(5)]-buttersäure-methylester

4,7 g (12,5 mMol) 4-[2-Benzolsulfonamido-indanyl-(5)]-oxo-buttersäure werden in 60 ml Eisessig mit 3 ml Perchlorsäure und 1 g 10%igem Palladium auf Kohle versetzt und bei 50°C unter 5 bar Wasserstoffdruck hydriert. Man dampft ein, versetzt den Rückstand mit Wasser und extrahiert das Reaktionsprodukt mit Essigester. Nach Eindampfen wird der Rückstand in 20 ml Methanol gelöst und bei -50°C mit 2 ml Thionylchlorid versetzt. Nach Stehen über Nacht dampft man erneut ein und chromatographiert den Rückstand mit Äthylenchlorid/Methanol = 98:2 an Kieselgel. Die Fraktionen mit einem RF-Wert von 0,7 (Kieselgel-Polygram-Platten; Chloroform/Methanol = 95:5) werden vereinigt und eingedampft.
Ausbeute: 1,4 g (30 % der Theorie).

Beispiel 37

4-[2-Benzolsulfonamido-indanyl-(5)]-buttersäure

Hergestellt analog Beispiel 35 aus 4-[2-Benzolsulfonamido-indanyl-(5)]-buttersäure-methylester durch Hydrolyse mit Natriumhydroxid.
Ausbeute: 81 % der Theorie,
Öl, RF-Wert: 0,5 (Kieselgel-Polygram-Platten; Cyclohexan/Essigester = 1:1 als Laufmittel)
NMR-Spektrum (in $CDCl_3$-$CD_3OD$):
CH und 10 aliphat. H: Multipletts bei 4.1 bzw. 1.8-3.3 ppm.

Beispiel 38

4-[2-p-Chlorbenzolsulfonamido-indanyl-(5)]-buttersäure-methylester

2,8 g (12 mMol) 4-[2-Amino-indanyl-(5)]-buttersäure-methylester werden in 40 ml Chloroform mit 1,6 g (16 mMol) Triäthylamin und anschließend mit 3,2 g (14,4 mMol) p-Chlor benzolsulfonsäurechlorid versetzt. Man rührt drei Stunden bei Raumtemperatur, versetzt mit Wasser, dampft die Chloroformphase ein und reinigt das Reaktionsprodukt durch Chromatographie an Kieselgel mit Chloroform.
Ausbeute: 4,0 g (81,7 % der Theorie)
Öl, RF-Wert: 0,7 (Kieselgel-Polygram-Platten der Fa. Merck; Cyclohexan-Essigester = 1:1 als Laufmittel).
IR-Spektrum (in Methylenchlorid):
NH bei 3360 $cm^{-1}$, $OCH_3$ bei 2840 $cm^{-1}$, Ester-CO bei 1735 $cm^{-1}$, $SO_2$ bei 1160 + 1340 $cm^{-1}$
Schmelzpunkt: 82-85°C (Methanol)
$C_{20}H_{22}ClNO_4S$ (407,91)
Ber.: C 58,89 H 5,43 N 3,43 Cl 8,69 S 7,86
Gef.: 58,67 5,18 3,35 8,76 7,98

Beispiel 39

4-[2-p-Chlorbenzolsulfonamido-indanyl-(5)]-buttersäure

Hergestellt analog Beispiel 35 aus 4-[2-p-Chlorbenzolsulfonamido-indanyl-(5)]-buttersäure-methylester durch Hydrolyse mit Natriumhydroxid.
Ausbeute: 72 % der Theorie,
Schmelzpunkt: 131-133°C (Essigester/Diisopropyläther)
$C_{19}H_{20}ClNO_4S$ (393,88)
Ber.: C 57,94 H 5,12 N 3,56 Cl 9,00 S 8,14
Gef.: 57,65 5,33 3,59 9,06 8,22

Beispiel 40

4-[2-p-Toluolsulfonamido-indanyl-(5)]-buttersäuremethylester

Hergestellt analog Beispiel 1 aus 4-[2-Amino-indanyl-(5)]-buttersäure-methylester durch p-Toluolsulfonsäurechlorid.
Ausbeute: 69 % der Theorie,
Schmelzpunkt: 97-99°C (Diisopropyläther)
$C_{21}H_{25}NO_4S$ (387,49)
Ber.: C 65,09 H 6,50 N 3,61 S 8,27
Gef.: 64,90 6,65 3,51 8,18

Beispiel 41

4-[2-p-Toluolsulfonamido-indanyl-(5)]-buttersäure

Hergestellt analog Beispiel 35 aus 4-[2-p-Toluolsulfonamido-indanyl-(5)]-buttersäure-methylester durch Hydrolyse mit Natriumhydroxid.
Ausbeute: 67 % der Theorie,
Schmelzpunkt: 130-132°C (Essigsäure/Diisopropyläther)
$C_{20}H_{23}NO_4S$ (373,47)
Ber.: C 64,32 H 6,21 N 3,75 S 8,58
Gef.: 64,40 6,41 3,65 8,53

Beispiel 42

4-[2-o-Toluolsulfonamido-indanyl-(5)]-buttersäuremethylester

Hergestellt analog Beispiel 1 aus 4-[2-Amino-indanyl-(5)]-buttersäure-methylester und o-Toluolsulfonsäurechlorid.
Ausbeute: 82 % der Theorie,
Öl, RF-Wert: 0,7 (auf Kieselgel-Polygram-Platten der Fa. Merck; Cyclohexan/Essigester = 1:1 als Laufmittel).
IR-Spektrum (in Methylenchlorid):
NH bei 3370 $cm^{-1}$, Ester-CO bei 1735 $cm^{-1}$, $SO_2$ bei 1160 und 1335 $cm^{-1}$

Beispiel 43

4-[2-o-Toluolsulfonamido-indanyl-(5)]-buttersäure

Hergestellt analog Beispiel 35 aus 4-[2-o-Toluolsulfonamid-indanyl-(5)]-buttersäure-methylester durch Hydrolye mit Natriumhydroxid.
Ausbeute: 90 % der Theorie,
Öl, RF-Wert: 0,5 (auf Kieselgel-Polygram-Platten der Fa. Merck; Cyclohexan/Essigester = 1:1 als Laufmittel).
$C_{20}H_{23}NO_4S$ (373,47)
Ber.: N 3,75 S 8,58
Gef.: 3,31 8,45

Beispiel 44

4-Hydroxy-4-[2-p-toluolsulfonamid-indanyl-(5)]-buttersäure

Man löst 400 mg (1,03 mMol) 4-[2-p-Toluolsulfonamido-indanyl-(5)]-4-oxo-buttersäure in 2 ml 2n Natronlauge, verdünnt mit 10 ml Wasser und versetzt mit 20 mg Natriumborhydrid. Man läßt über Nacht stehen, säuert an und extrahiert das Reaktionsprodukt mit Essigester. Der Eindampfrückstand wird mit Petroläther verrieben. Man erhält 400 mg (100 % der Theorie) eines kristallinen Materials, welches ab 60°C sintert.
RF-Wert: 0,3 (Kieselgel-Polygram-Platten der Firma Merck; Toluol/Dioxan/Äthanol/Eisessig = 90:10:10:6 als Laufmittel).
IR-Spektrum (in Methylenchlorid):
OH bei 3600 $cm^{-1}$, NH bei 3360 $cm^{-1}$, CO bei 1780, 1750, 1710 $cm^{-1}$,
$SO_2$ bei 1160 und 1340 $cm^{-1}$

Beispiel 45

[2-p-Chlorbenzolsulfonamido-indanyl-(5)]-essigsäure-methylester

Hergestellt analog Beispiel 5 aus [2-Amino-indanyl-(5)]-essigsäure-methylester-hydrochlorid und p-Chlorbenzolsulfonsäurechlorid in Chloroform in Gegenwart von Triäthylamin. Reinigung durch Säulenchromatographie an Kieselgel mit Äthylenchlorid-Methanol = 98:2.
Ausbeute: 20 % der Theorie,
Öl, RF-Wert: 0.8 (Kieselgel-Polygram-Platten; Chloroform-Methanol = 95/5 als Laufmittel).
IR-Spektrum (in Methylenchlorid):
NH bei 3360 $cm^{-1}$, CO bei 1740 $cm^{-1}$, $SO_2$ bei 1160 und 1340 $cm^{-1}$

Beispiel 46

[2-p-Chlorbenzolsulfonamido-indanyl-(5)]-essigsäure

Hergestellt analog Beispiel 2 aus [2-p-Chlorbenzolsulfonamido-indanyl-(5)]-essigsäure-methylester durch Hydrolyse mit Natriumhydroxid.
Ausbeute: 67 % der Theorie,
Schmelzpunkt: 156-158°C (aus Toluol)
$C_{17}H_{16}ClNO_4S$ (365,83)
Ber.: C 55,81 H 4,41 N 3,83 Cl 9,69 S 8,76
Gef.: 56,47 4,45 4,00 9,94 8,98

Beispiel 47

[2-p-Toluolsulfonamido-indanyl-(5)]-essigsäure-methylester

Hergestellt analog Beispiel 5 aus [2-Amino-indanyl-(5)]-essigsäure-methylester-hydrochlorid und p-Toluolsulfochlorid in Chloroform in Gegenwart von Triäthylamin. Reinigung durch Säulenchromatographie an Kieselgel mit Äthylenchlorid-Methanol = 98/2.
Ausbeute: 63 % der Theorie,
Öl, RF-Wert: 0,8 (Kieselgel-Polygram-Platten; Chloroform-Methanol = 95/5 als Laufmittel).
IR-Spektrum (in Methylenchlorid):
NH bei 3360 $cm^{-1}$, $OCH_3$ bei 2840 $cm^{-1}$, CO bei 1735 $cm^{-1}$, $SO_2$ bei 1160 und 1340 $cm^{-1}$

Beispiel 48

[2-p-Toluolsulfonamido-indanyl-(5)]-essigsäure

Hergestellt analog Beispiel 2 aus [2-p-Toluolsulfonamido-indanyl-(5)]-essigsäure-methylester durch Hydrolyse mit Natriumhydroxid.
Ausbeute: 71 % der Theorie,
Schmelzpunkt: 143-145°C (aus Toluol)
$C_{18}H_{19}NO_4S$ (345,41)
Ber.: C 62,59 H 5,54 N 4,06 S 9,28
Gef.: 62,82 5,41 4,03 9,39

Beispiel 49

[2-o-Toluolsulfonamido-indanyl-(5)]-essigsäure-methylester

Hergestellt analog Beispiel 5 aus [2-Amino-indanyl-(5)]-essigsäure-methylester-hydrochlorid und o-Toluolsulfonsäurechlorid in Chloroform in Gegenwart von Triäthylamin. Reinigung durch Säulenchromatographie an Kieselgel mit Äthylenchlorid-Methanol = 98:2.
Ausbeute: 10 % der Theorie,
Öl, RF-Wert: 0,85 (Kieselgel-Polygram-Platten; Chloroform-Methanol = 95/5 als Laufmittel).
IR-Spektrum (in Methylenchlorid):
NH bei 3370 $cm^{-1}$, $OCH_3$ bei 2840 $cm^{-1}$, CO bei 1740 $cm^{-1}$, $SO_2$ bei 1160 und 1335 $cm^{-1}$

Beispiel 50

[2-o-Toluolsulfonamido-indanyl-(5)]-essigsäure

Hergestellt analog Beispiel 2 aus [2-o-Toluolsulfonamido-indanyl-(5)]-essigsäure-methylester durch Hydrolyse mit Natriumhydroxid.
Ausbeute: 30 % der Theorie,
Harz, RF-Wert: 0,5 (Kieselgelplatten F 254 der Fa. Merck, Darmstadt; Toluol-Dioxan-Äthanol-Eisessig = 90:10:10:6 als Laufmittel).
$C_{18}H_{19}NO_4S$ (345,41)
Ber.: C 62,59 H 5,54 N 4,06 S 9,28
Gef.: 62,80 5,62 4,19 9,05

Beispiel 51

[2-p-Methoxybenzolsulfonamido-indanyl-(5)]-essigsäure-methylester

Hergestellt analog Beispiel 5 aus [2-Amino-indanyl-(5)]-essigsäure-methylester-hydrochlorid und p-Methoxybenzolsulfonsäurechlorid in Chloroform in Gegenwart von Triäthylamin. Reinigung durch Säulenchromatographie an Kieselgel mit Äthylenchlorid-Methanol = 98/2.
Ausbeute: 56 % der Theorie,
Öl, RF-Wert: 0,7 (Kieselgel-Polygram-Platten; Chloroform-Methanol = 95/5 als Laufmittel).
IR-Spektrum (in Methylenchlorid):
NH bei 3360 $cm^{-1}$, $OCH_3$ bei 2840 $cm^{-1}$, $SO_2$ bei 1150 und 1340 $cm^{-1}$

Beispiel 52

[2-p-Methoxybenzolsulfonamido-indanyl-(5)]-essigsäure

Hergestellt analog Beispiel 2 aus [2-p-Methoxybenzolsulfonamido-indanyl-(5)]-essigsäure-methylester durch Hydrolyse mit Natriumhydroxid.
Ausbeute: 73 % der Theorie,
Schmelzpunkt: 156-158°C (aus Essigsäureäthylester-Diisopropylether).
$C_{18}H_{19}NO_5S$ (361,41)
Ber.: C 59,82 H 5,30 N 3,88 S 8,87
Gef.: 60,13 5,24 3,88 8,75

Beispiel 53

[2-o-Methoxybenzolsulfonamido-indanyl-(5)]-essigsäure-methylester

Hergestellt analog Beispiel 5 aus [2-Amino-indanyl-(5)]-essigsäure-methylester-hydrochlorid und o-Methoxybenzolsulfonsäurebromid in Chloroform in Gegenwart von Triäthylamin. Reinigung durch Säulenchromatographie an Kieselgel mit Äthylenchlorid-Methanol = 99/1 als Laufmittel.
Ausbeute: 75 % der Theorie,
Öl, RF-Wert: 0,7 (Kieselgel-Polygram-Platten; Chloroform-Methanol = 95/5 als Laufmittel).
IR-Spektrum (in Methylenchlorid):
NH bei 3350 $cm^{-1}$, $OCH_3$ bei 2840 $cm^{-1}$, $SO_2$ bei 1160 und 1340 $cm^{-1}$

Beispiel 54

[2-o-Methoxybenzolsulfonamido-indanyl-(5)]-essigsäure

Hergestellt analog Beispiel 2 aus [2-o-Methoxybenzolsulfonamido-indanyl-(5)]-essigsäure-methylester durch Hydrolyse mit Natriumhydroxid.
Ausbeute: 60 % der Theorie,
Schmelzpunkt: 134-136°C (aus Essigsäureäthylester-Diisopropylether).
$C_{18}H_{19}NO_5S$ (361,41)
Ber.: C 59,82 H 5,30 N 3,88 S 8,87
Gef.: 59,80 5,37 3,79 8,73

Beispiel 55

[2-(2,5-Dichlorbenzolsulfonyl)amido-indanyl-(5)]-essigsäure-methylester

Hergestellt analog Beispiel 5 aus [2-Amino-indanyl-(5)]-essigsäure-methylester-hydrochlorid und 2,5-Dichlorbenzolsulfonsäurechlorid in Chloroform in Gegenwart von Triäthylamin. Reinigung durch Säulenchromatographie an Kieselgel mit Äthylenchlorid-Methanol = 99/1.
Ausbeute: 64 % der Theorie,
Öl, RF-Wert: 0,8 (Kieselgel-Polygram-Platten; Chloroform-Methanol = 95/5 als Laufmittel).
IR-Spektrum (in Methylenchlorid):
NH bei 3360 $cm^{-1}$, CO bei 1740 $cm^{-1}$, $SO_2$ bei 1165 und 1350 $cm^{-1}$

Beispiel 56

[2-(2,5-Dichlorbenzolsulfonyl)amido-indanyl-(5)]-essigsäure

Hergestellt analog Beispiel 2 aus [2-(2,5-Dichlorbenzolsulfonyl)amido-indanyl-(5)]-essigsäure-methylester durch Hydrolyse mit Natriumhydroxid.
Ausbeute: 57 % der Theorie,
Schmelzpunkt: 172-174°C (aus Essigsäureäthylester-Diisopropylether/Petrolether).
$C_{17}H_{15}Cl_2NO_4S$ (400,28)
Ber.: C 51,01 H 3,78 Cl 17,71 N 3,50 S 8,01
Gef.: 51,03 3,87 17,81 3,52 8,03

Beispiel 57

[2-p-Fluorbenzolsulfonamido-indanyl-(5)]-essigsäure-methylester

Hergestellt analog Beispiel 5 aus [2-Amino-indanyl-(5)]-essigsäure-methylester-hydrochlorid und p-Fluorbenzolsulfonsäurechlorid in Chloroform in Gegenwart von Triäthylamin. Reinigung durch Säulenchromatographie an Kieselgel mit Äthylenchlorid-Methanol = 98/2.
Ausbeute: 54 % der Theorie,
Öl, RF-Wert: 0,8 (Kieselgel-Polygram-Platten; Chloroform-Methanol = 95/5 als Laufmittel).
IR-Spektrum (in Methylenchlorid):
NH bei 3360 $cm^{-1}$, CO bei 1735 $cm^{-1}$, $SO_2$ bei 1150 und 1340 $cm^{-1}$

Beispiel 58

[2-p-Fluorbenzolsulfonamido-indanyl-(5)]-essigsäure

Hergestellt analog Beispiel 2 aus [2-p-Fluorbenzolsulfonamido-indanyl-(5)]-essigsäure-methylester durch Hydrolyse mit Natriumhydroxid.
Ausbeute: 43 % der Theorie,
Schmelzpunkt: 129,5-131,5°C (aus Essigsäureäthylester-Diisopropylether).
$C_{17}H_{16}FNO_4S$ (349,38)
Ber.: C 58,44 H 4,61 N 4,01 S 9,18
Gef.: 58,60 4,63 3,95 9,37

Beispiel 59

2-[2-p-Toluolsulfonamido-indanyl-(5)]-propionsäure-methylester

3,2 g (0,0125 Mol) 2-[2-Amino-indanyl-(5)]-propionsäure-methylester-hydrochlorid und 2,7 g (0,014 Mol) p-Toluolsulfonsäurechlorid werden in Chloroform gelöst und unter Rühren tropfenweise mit 2,53 g (0,025 Mol) Triäthylamin versetzt. Man läßt über Nacht bei Raumtemperatur stehen. Anschließend versetzt man mit Wasser, trennt die Chloroformphase ab und dampft im Vakuum ein. Der verbleibende Rückstand (4,6 g) wird an 300 g Kieselgel mit Äthylenchlorid/Äthanol = 98:2 chromatographiert.
Ausbeute: 1,2 g (26 % der Theorie),
Öl, Rf-Wert = 0,2 (auf Kieselgel-Polygramplatten SIL G/UV der Fa. Macherey-Nagel, Düren; Laufmittel: Äthylenchlorid).

Beispiel 60

2-[2-p-Toluolsulfonamido-indanyl-(5)]-propionsäure

1,2 g 2-[2-p-Toluolsulfonamido-indanyl-(5)]-propionsäure-methylester werden in 12 ml Äthanol mit 1 ml 15 N Natriumhydroxid-Lösung versetzt und 0,5 Stunden gekocht. Man dampft ein, löst den Rückstand in Wasser und fällt das Reaktionsprodukt mit Salzsäure aus.
Ausbeute: 1,0 g (87 % der Theorie),
langsam erstarrendes Öl.
$C_{19}H_{21}NO_4S$ (359,44)
Ber.: C 63,49 H 5,89 N 3,90 S 8,92
Gef.: 63,38 6,19 3,92 9,22
IR-Spektrum (in Methylenchlorid):
NH bei 3360 $cm^{-1}$, CO bei 1715 und 1750 $cm^{-1}$,
$SO_2$ bei 1160 und 1340 $cm^{-1}$.

Beispiel 61

2-[2-p-Chlorbenzolsulfonamido-indanyl-(5)]-propionsäure-methylester

Hergestellt analog Beispiel 59 aus 2-[2-Amino-indanyl-(5)]-propionsäure-methylester-hydrochlorid und p-Chlorbenzolsulfonsäurechlorid in Chloroform in Gegenwart von Triäthylamin.
Ausbeute: 15 % der Theorie,
Öl, Rf-Wert = 0,2 (auf Kieselgel-Polygramplatten SIL G/UV der Fa. Macherey-Nagel, Düren; Laufmittel: Äthylenchlorid).

Beispiel 62

2-[2-p-Chlorbenzolsulfonamido-indanyl-(5)]-propionsäure

Hergestellt analog Beispiel 60 aus 2-[2-p-Chlorbenzolsulfonamido-indanyl-(5)]-propionsäure-methylester durch Hydrolyse mit Natriumhydroxid.
Ausbeute: 69 % der Theorie,
Schmelzpunkt: 87-90 °C
$C_{18}H_{18}ClNO_4S$ (379,86)
Ber.: C 56,92 H 4,78 N 3,69 Cl 9,33 S 8,44
Gef.: 56,95 4,71 3,64 9,41 8,27

Beispiel 63

2-Carbäthoxy-4-[2-p-toluolsulfonamido-indanyl-(5)]-4-oxo-buttersäure-äthylester

15,2 g (0,095 Mol) Malonsäure-diäthylester in 25 ml Dimethylsulfoxid werden mit 10,6 g (0,095 Mol) Kalium-tert.buta nolat und anschließend mit 23,0 g (0,063 Mol) [2-p-Toluolsulfonamido-indanyl-(5)]-chlormethyl-keton versetzt. Man läßt über Nacht stehen, neutralisiert mit Eisessig und versetzt mit Wasser und Petroläther. Das ausgefallene Reaktionsprodukt wird abgetrennt und in Essigester gelöst. Nach Waschen der organischen Phase mit Wasser und Trocknen wird eingedampft und der erhaltene Rückstand durch Chromatographie an 600 g Kieselgel mit Äthylenchlorid/Äthanol = 98:2 gereinigt.
Ausbeute: 20,2 g (65,5 % der Theorie),
Schmelzpunkt: 101-103 °C (Essigester/Petroläther)
$C_{25}H_{29}NO_7S$ (487,57)
Ber.: C 61,59 H 5,99 N 2,87 S 6,58
Gef.: 61,29 6,00 2,98 6,42

Analog wird dargestellt:
2-Carbäthoxy-4-[2-p-chlorbenzolsulfonamido-indanyl-(5)]-4-oxo-buttersäure-äthylester
Ausbeute: 96 % der Theorie,
Öl, Rf-Wert: 0,4 (Kieselgel-Polygram-Platten SIL G/UV der Fa. Macherey-Nagel, Düren; Laufmittel: Cyclohexan/Essigester = 3:1).

Beispiel 64

4-Carbäthoxy-6-[2-p-chlorbenzolsulfonamido-indanyl-(5)]-4,5-dihydropyridazin-3(2H)-on

Hergestellt analog Beispiel 26 aus 2-Carbäthoxy-4-[2-p-chlorbenzolsulfonamido-indanyl-(5)]-4-oxobuttersäure-äthylester und Hydrazin durch Kochen in Eisessig.
Ausbeute: 38 % der Theorie,
Schmelzpunkt: 174-177°C (Eisessig)
$C_{22}H_{22}ClN_3O_5S$ (475,95)
Ber.: C 55,52 H 4,66 N 8,33 Cl 7,45 S 6,74
Gef.: 55,53 4,59 8,50 7,36 6,64
IR-Spektrum (in KBr):
Ester-CO 1725 $cm^{-1}$, $SO_2NH$ bei 1160 und 1340 $cm^{-1}$.

Beispiel 65

4-Carbäthoxy-6-[2-p-toluolsulfonamido-indanyl-(5)]-4,5-dihydro-pyridazin-3(2H)-on

Hergestellt analog Beispiel 26 aus 2-Carbäthoxy-4-[2-p-toluolsulfonamido-indanyl-(5)]-4-oxobuttersäure-äthylester mit Hydrazin durch Kochen in Eisessig.
Ausbeute: 82 % der Theorie,
Schmelzpunkt: 187-189°C (Eisessig)
$C_{23}H_{25}N_3O_5S$ (455,53)
Ber.: C 60,64 H 5,53 N 9,22 S 7,04
Gef.: 60,25 5,55 9,66 7,10
IR-Spektrum (in KBr):
Ester-CO 1740 $cm^{-1}$, Lactam-CO bei 1665 $cm^{-1}$, C=N bei 1615 $cm^{-1}$, $SO_2NH$ bei 1150 und 1330 $cm^{-1}$.

Beispiel 66

6-[2-p-Toluolsulfonamido-indanyl-(5)]-4,5-dihydropyridazin-3-(2H)-on

2,3 g (5 mMol) 4-Carbäthoxy-6-[2-p-toluolsulfonamido-indanyl-(5)]-4,5-dihydro-pyridazin-3(2H)-on werden in 40 ml Äthanol mit 600 mg (15 mMol) Natriumhydroxid in 2 ml Wasser versetzt und zur Hydrolyse über Nacht gerührt. Das beim Ansäuern ausfallende Reaktionsprodukt wird abgesaugt und in Eisessig 10 Minuten lang zum Sieden erhitzt. Beim Abkühlen kristallisiert die gewünschte Verbindung aus.
Ausbeute: 1,8 g (93 % der Theorie),
Schmelzpunkt: 185-187°C.

Beispiel 67

6-[2-p-Chlorbenzolsulfonamido-indanyl-(5)]-4,5-dihydropyridazin-3(2H)-on

Hergestellt analog Beispiel 66 aus 4-Carbäthoxy-6-[2-p-chlorbenzolsulfonamido-indanyl-(5)]-4,5-dihydropyridazin-3(2H)-on durch Hydrolyse und Decarboxylierung.
Ausbeute: 67 % der Theorie,
Schmelzpunkt: 203-205°C.

Beispiel I

Tabletten mit 100 mg 4-[2-p-Toluolsulfonamido-indanyl-(5)]-4-oxo-buttersäure

Zusammensetzung:

1 Tablette enthält:

| | |
|---|---|
| Wirkstoff | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

Herstellungsverfahren:

Wirkstoff, Milchzucker und Stärke werden gemischt und mit einer wäßrigen Lösung des Polyvinylpyrrolidons gleichmäßig befeuchtet. Nach Siebung der feuchten Massen (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.
Tablettengewicht: 220 mg
Durchmesser: 9 mm, biplan mit beidseitiger Facette und einseitiger Teilkerbe.

Beispiel II

Hartgelatine-Kapseln mit 150 mg 4-[2-p-Toluolsulfonamido-indanyl-(5)]-4-oxo-buttersäure

1 Kapsel enthält:

| | | |
|---|---|---|
| Wirkstoff | | 150,0 mg |
| Maisstärke getr. | ca. | 180,0 mg |
| Milchzucker pulv. | ca. | 87,0 mg |
| Magnesiumstearat | | 3,0 mg |
| | ca. | 320,0 mg |

Herstellung:

Der Wirkstoff wird mit den Hilfsstoffen vermengt, durch ein Sieb von 0,75 mm-Maschenweite gegeben und in einem geeigneten Gerät homogen gemischt.
Die Endmischung wird in Hartgelatine-Kapseln der Größe 1 abgefüllt.
Kapselfüllung: ca. 320 mg
Kapselhülle: Hartgelatine-Kapsel Größe 1.

Beispiel III

Suppositorien mit 150 mg 4-[2-p-Toluolsulfonamido-indanyl-(5)]-4-oxo-buttersäure

1 Zäpfchen enthält:

| | |
|---|---|
| Wirkstoff | 150,0 mg |
| Polyäthylenglykol (M.G. 1500) | 550,0 mg |
| Polyäthylenglykol (M.G. 6000) | 460,0 mg |
| Polyäthylensorbitanmonostearat | 840,0 mg |
| | 2 000,0 mg |

Herstellung:

Nach dem Aufschmelzen der Suppositorienmassen wird der Wirkstoff darin homogen verteilt und die Schmelze in vorgekühlte Formen gegossen.

Beispiel IV

Suspensionen mit 50 mg 4-[2-p-Toluolsulfonamido-indanyl-(5)]-4-oxo-buttersäure

100 ml Suspension enthalten:

| | | |
|---|---|---|
| Wirkstoff | | 1,0 g |
| Carboxymethylcellulose-Na-Salz | | 0,2 g |
| p-Hydroxybenzoesäuremethylester | | 0,05 g |
| p-Hydroxybenzoesäurepropylester | | 0,01 g |
| Glycerin | | 5,0 g |
| Sorbitlösung 70%ig | | 50,0 g |
| Aroma | | 0,3 g |
| Wasser dest. | ad | 100 ml |

Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe der Sorbitlösung und des Aromas wird die Suspension zur Entlüftung unter Rühren evakuiert.
5 ml Suspension enthalten 50 mg Wirkstoff.

Beispiel V

Tabletten mit 150 mg [2-p-Chlorbenzolsulfonamido-indanyl-(5)]-essigsäure

Zusammensetzung:
1 Tablette enthält:

| | |
|---|---|
| Wirksubstanz | 150,0 mg |
| Milchzucker pulv. | 89,0 mg |
| Maisstärke | 40,0 mg |
| Kolloidale Kieselsäure | 10,0 mg |
| Polyvinylpyrrolidon | 10,0 mg |
| Magnesiumstearat | 1,0 mg |
| | 300,0 mg |

Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen. Das bei 45°C getrocknete Granulat wird nochmals durch dasselbe Sieb gerieben und mit der angegebenen Menge Magnesiumstearat gemischt. Aus der Mischung werden Tabletten gepreßt.
Tablettengewicht: 300 mg
Stempel: 10 mm, flach

Beispiel VI

Filmtabletten mit 75 mg [2-p-Chlorbenzolsulfonamido-indanyl-(5)]-essigsäure

| | |
|---|---|
| 1 Tablettenkern enthält: | |
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm Maschenweite gerieben und mit der restlichen Menge Magnesiumstearat vermischt. Dieses Granulat wird auf einer Tablettiermaschine zu Tabletten mit der gewünschten Form gepreßt.

Kerngewicht: 230 mg

Stempel: 9 mm, gewölbt

Die so hergestellten Tablettenkerne werden mit einem Film überzogen, der im wesentlichen aus Hydroxypropylmethylcellulose besteht. Die fertigen Filmtabletten werden mit Bienenwachs geglänzt.

Filmtablettengewicht: 245 mg

Selbstverständlich können alle übrigen Verbindungen der allgemeinen Formel I als Wirkstoffe in den vorstehenden galenischen Zubereitungen eingesetzt werden.

**Ansprüche**

1. Neue Benzolsulfonamido-indanylverbindungen der allgemeinen Formel

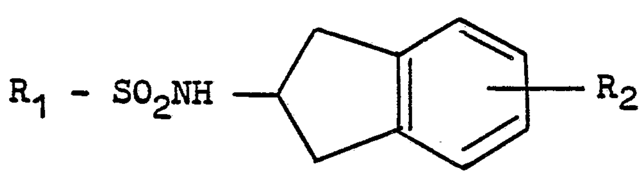

(I)

in der

$R_1$ eine gegebenenfalls durch ein Halogenatom, eine Methyl-oder Methoxygruppe mono-oder disubstituierte Phenylgruppe, wobei die Substituenten gleich oder verschieden sein können, und

$R_2$ eine gegebenenfalls über eine geradkettige oder verzweigte Alkylengruppe mit 1 bis 5 Kohlenstoffatomen oder über eine Alkenylengruppe mit 2 bis 5 Kohlenstoffatomen gebundene Hydroxycarbonyl-oder Alkoxycarbonylgruppe mit insgesamt 2 bis 4 Kohlenstoffatomen, wobei jeweils eine Methylengruppe der vorstehend erwähnten Alkylen-oder Alkenylenreste, welche mit dem Indanylrest verknüpft sein muß, durch

eine Hydroxymethylen-oder Carbonylgruppe ersetzt sein kann und gleichzeitig die Methylengruppe, über die die vorstehend erwähnte Alkoxycarbonylgruppe gebunden ist, durch eine weitere Alkoxycarbonylgruppe mit insgesamt 2 bis 4 Kohlenstoffatomen substituiert sein kann, oder eine 4,5-Dihydro-pyridazin-3(2H)-on-6-yl-oder Pyridazin-3(2H)-on-6-yl-gruppe, welche in 4-oder 5-Stellung durch eine Alkylgruppe mit 1 bis 3 Kohlenstoffatomen und/oder in 4-Stellung durch eine Alkoxycarbonylgruppe mit insgesamt 2 bis 4 Kohlenstoffatomen substituiert sein können bedeuten, deren Enantiomere und deren Salze mit anorganischen oder organischen Basen, falls $R_2$ eine Hydroxycarbonylgruppe enthält.

2. Neue Benzolsulfonamido-indanylverbindungen der allgemeinen Formel I gemäß Anspruch 1, in der $R_1$ eine gegebenenfalls durch eine Methyl-oder Methoxygruppe substituierte Phenylgruppe oder eine durch ein Fluor-, Chlor-oder Bromatom mono-oder disubstituierte Phenylgruppe und

$R_2$ eine gegebenenfalls über eine geradkettige oder verzweigte Alkylengruppe mit 1 bis 4 Kohlenstoffatomen oder über eine geradkettige oder verzweigte Alkenylengruppe mit 2 bis 4 Kohlenstoffatomen gebundene Hydroxycarbonyl-oder Alkoxycarbonylgruppe mit insgesamt 2 oder 3 Kohlenstoffatomen, wobei jeweils eine Methylengruppe der vorstehend erwähnten Alkylen-oder Alkenylengruppen, welche mit dem Indanylrest verknüpft sein muß, durch eine Hydroxymethylen-oder Carbonylgruppe ersetzt sein kann, oder eine gegebenenfalls in 5-Stellung durch eine Methylgruppe substituierte 4,5-Dihydro-pyridazin-3(2H)-on-6-yl-oder Pyridazin-3(2H)-on-6-yl-gruppe, welche zusätzlich in 4-Stellung durch eine Alkoxycarbonylgruppe mit insgesamt 2 oder 3 Kohlenstoffatomen substituiert sein kann, bedeuten, deren Enantiomere und deren Salze mit anorganischen oder organischen Basen, falls $R_2$ eine Hydroxycarbonylgruppe enthält.

3. Neue Benzolsulfonamido-indanylverbindungen der allgemeinen Formel I gemäß Anspruch 1, in der $R_1$ eine gegebenenfalls durch ein Fluor-oder Chloratom, durch eine Methyl-oder Methoxygruppe substituierte Phenylgruppe und

$R_2$ eine Hydroxycarbonylmethyl-, 4-Hydroxycarbonyl-n-propyl-, 3-Hydroxycarbonyl-n-propanon-(1)-yl-, 4,5-Dihydro-pyridazin-3(2H)-on-6-yl-oder 4,5-Dihydro-5-methyl-pyridazin-3(2H)-on-yl-gruppe bedeuten, deren Enantiomere und deren Salze mit anorganischen oder organischen Basen, falls $R_2$ eine Hydroxycarbonylgruppe enthält.

4. 4-[2-p-Toluolsulfonamido-indanyl-(5)]-4-oxo-buttersäure und deren Additionssalze mit anorganischen Basen.

5. [2-p-Chlorbenzolsulfonamido-indanyl-(5)]-essigsäure und deren Additionssalze mit anorganischen Basen.

6. Physiologisch verträgliche Additionssalze der Verbindungen gemäß den Ansprüchen 1 bis 5 mit anorganischen oder organischen Basen.

7. Arzneimittel, enthaltend als Wirkstoff eine Verbindung gemäß den Ansprüchen 1 bis 5 oder dessen physiologisch verträgliches Additionssalz gemäß Anspruch 6 neben gegebenenfalls einem oder mehreren inerten Trägerstoffen und/oder Verdünnungsmitteln.

8. Verwendung einer Verbindung gemäß den Ansprüchen 1 bis 5 oder dessen physiologisch verträgliches Additionssalz gemäß Anspruch 6 zur Herstellung eines Arzneimittels zur Behandlung und zur Prophylaxe thrombo-embolischer Erkrankungen, zur Prophylaxe der Arteriosklerose und zur Metastasenprophylaxe.

9. Verfahren zur Herstellung eines Arzneimittels gemäß Anspruch 7, dadurch gekennzeichnet, daß auf nicht-chemischem Wege eine Verbindung gemäß den Ansprüchen 1 bis 5 oder dessen physiologisch verträgliches Additionssalz gemäß Anspruch 6 in einen oder mehrere inerte übliche Trägerstoffe und/oder Verdünnungsmittel eingearbeitet wird.

10. Verfahren zur Herstellung der Verbindungen gemäß den Ansprüchen 1 bis 6, dadurch gekennzeichnet, daß

a.) eine Verbindung der allgemeinen Formel

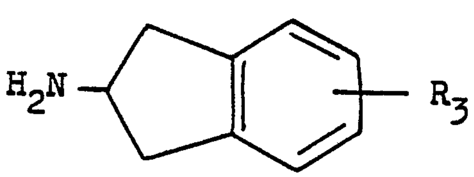

(II)

in der

$R_3$ die für $R_2$ in den Ansprüchen 1 bis 5 erwähnten Bedeutungen aufweist, wobei jedoch eine Hydroxygruppe im Rest $R_2$ durch eine hydrolytisch oder hydrogenolytisch abspaltbare Schutzgruppe wie eine Alkoxy-oder Benzyloxygruppe geschützt sein kann, mit einem Phenylsulfonsäurederivat der allgemeinen

Formel

$R_1$ -$SO_2X$ ,(III)

in der

$R_1$ wie in den Ansprüchen 1 bis 5 definiert ist und

X eine nucleophile Austrittsgruppe wie ein Halogenatom oder eine Alkoxygruppe darstellt, acyliert wird und gegebenenfalls anschließend ein verwendeter Schutzrest abgespalten wird oder

b.) zur Herstellung von Verbindungen der allgemeinen Formel I, in der $R_2$ eine Hydroxycarbonylgruppe darstellt oder enthält, ein Schutzrest von einer Verbindung der allgemeinen Formel

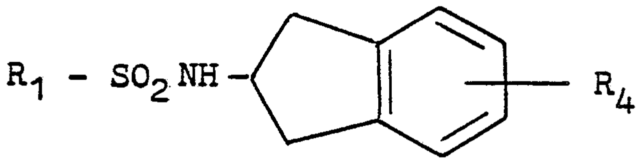

(IV)

in der

$R_1$ wie in den Ansprüchen 1 bis 5 definiert ist und

$R_4$ die für $R_2$ in den Ansprüchen 1 bis 5 erwähnten Bedeutungen besitzt, wobei jedoch die Carboxygruppe durch einen hydrolytisch, thermolytisch oder hydrogenolytisch abspaltbare Schutzgruppe geschützt ist oder ein funktionelles Derivat der Carboxygruppe darstellt und/oder der Rest $R_2$ eine durch einen Schutzrest geschützte Hydroxygruppe enthält, abgespalten wird oder

c.) zur Herstellung von Verbindungen der allgemeinen Formel I, in der $R_2$ benachbart zum Indanylrest eine Carbonylgruppe enthält, eine Verbindung der allgemeinen Formel

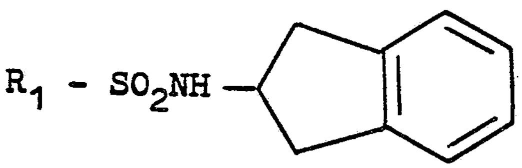

(V)

in der

$R_1$ wie in den Ansprüchen 1 bis 5 definiert ist, mit einer Verbindung der allgemeinen Formel

Y - $R_5$ ,(VI)

in der

$R_5$ die für $R_2$ in den Ansprüchen 1 bis 5 erwähnten Bedeutungen besitzt, wobei jedoch $R_2$ benachbart zu Y eine Carbonylgruppe enthalten muß und gleichzeitig eine gegebenenfalls vorhandene Hydroxycarbonylgruppe durch einen hydrolytisch oder hydrogenolytisch abspaltbaren Schutzrest wie eine Alkoxy-oder Benzylgruppe geschützt sein kann, und

Y eine nucleophile Austrittsgruppe wie ein Halogenatom darstellen, oder dessen Anhydrid in Gegenwart einer Lewis-Säure acyliert wird und gegebenenfalls anschließend ein verwendeter Schutzrest abgespalten wird oder

d.) zur Herstellung von Verbindungen der allgemeinen Formel I, in der $R_2$ benachbart zum Indanylrest eine Hydroxymethylen-oder Methylengruppe enthält, eine Verbindung der allgemeinen Formel

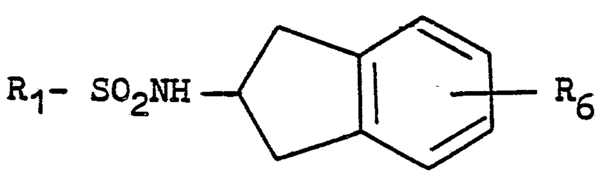

(VII)

in der

$R_1$ wie in den Ansprüchen 1 bis 5 definiert ist und

$R_6$ die für $R_2$ in den Ansprüchen 1 bis 5 erwähnten Bedeutungen aufweist, wobei jedoch $R_2$ benachbart zum Indanylrest eine Carbonylgruppe enthalten muß, reduziert wird oder

e.) zur Herstellung von Verbindungen der allgemeinen Formel I, in der $R_2$ einen der in den Ansprüchen 1

bis 5 erwähnten gesättigten Reste darstellt, eine Verbindung der allgemeinen Formel

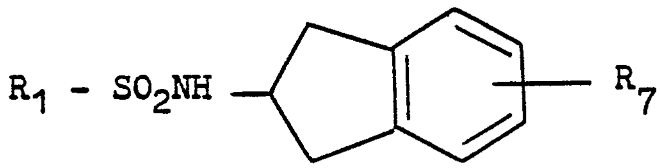

(VIII)

in der
$R_1$ wie in den Ansprüchen 1 bis 5 definiert ist und
$R_7$ einen der für $R_2$ in den Ansprüchen 1 bis 5 erwähnten ungesättigen Rest darstellt, hydriert wird oder
f.) zur Herstellung von Verbindungen der allgemeinen Formel I, in der $R_2$ einen Pyridazinonring darstellt, eine Verbindung der allgemeinen Formel

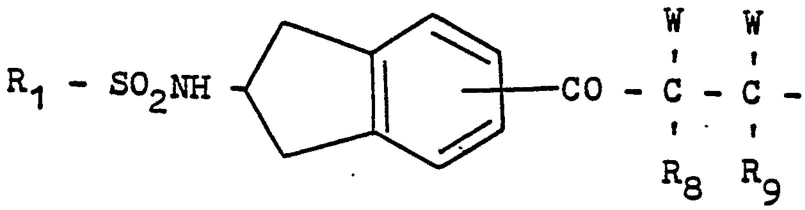

(IX)

in der
$R_1$ wie in den Ansprüchen 1 bis 5 definiert ist,
$R_8$ ein Wasserstoffatom oder eine Alkylgruppe mit 1 bis 3 Kohlenstoffatomen,
$R_9$ ein Wasserstoffatom, eine Alkylgruppe mit 1 bis 3 Kohlenstoffatomen oder eine Alkoxycarbonylgruppe mit insgesamt 2 bis 4 Kohlenstoffatomen, wobei jedoch nur einer der Reste $R_8$ oder $R_9$ eine Alkylgruppe mit 1 bis 3 Kohlenstoffatomen darstellen kann, und
W jeweils ein Wasserstoffatom oder zusammen eine weitere Bindung darstellen, oder deren reaktionsfähige Derivate wie deren Ester, Amide oder Halogenide mit Hydrazin umgesetzt wird oder
g.) zur Herstellung von Verbindungen der allgemeinen Formel I, in der $R_2$ die Carboxygruppe darstellt, eine Verbindung der allgemeinen Formel

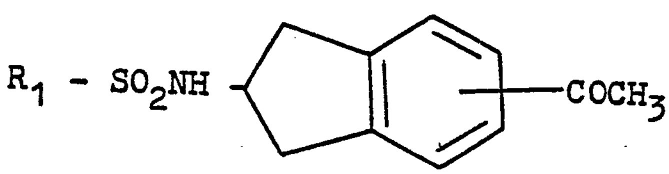

(X)

in der
$R_1$ wie in den Ansprüchen 1 bis 5 definiert ist, mit einem Hypohalogenit umgesetzt wird oder
h.) zur Herstellung von Verbindungen der allgemeinen Formel I, in der $R_2$ die Hydroxycarbonylmethylgruppe darstellt, eine Verbindung der allgemeinen Formel

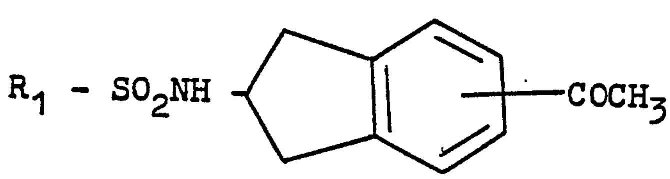

(X)

in der
$R_1$ wie in den Ansprüchen 1 bis 5 definiert ist, mit Schwefel in Gegenwart eines Amins umgesetzt und anschließend die so erhaltene Verbindung verseift wird oder
i.) zur Herstellung von Verbindungen der allgemeinen Formel I, in der $R_2$ eine 2-Hydroxycarbonyl-äthenyl- oder 2-Alkoxycarbonyl-äthenylgruppe darstellt, eine Verbindung der allgemeinen Formel

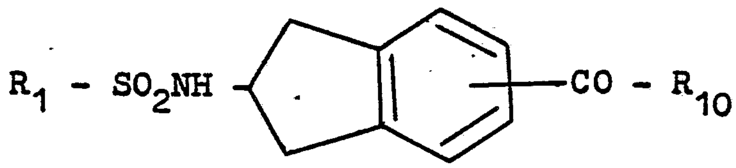

(XI)

in der
$R_1$ wie in den Ansprüchen 1 bis 5 definiert ist und
$R_{10}$ ein Wasserstoffatom oder eine Alkylgruppe mit 1 bis 3 Kohlenstoffatomen darstellt, mit einem entsprechenden Phosphonoessigsäure-trialkylester umgesetzt und die so erhaltene Verbindung erforderlichenfalls anschließend hydrolysiert wird oder
k) zur Herstellung von Verbindungen der allgemeinen Formel I, in der $R_2$ einen gegebenenfalls in 4-oder 5-Stellung durch eine Alkylgruppe substituierten 4,5-Dihydro-pyridazin-3-on-oder Pyridazin-3-on-Ring darstellt, eine Verbindung der allgemeinen Formel

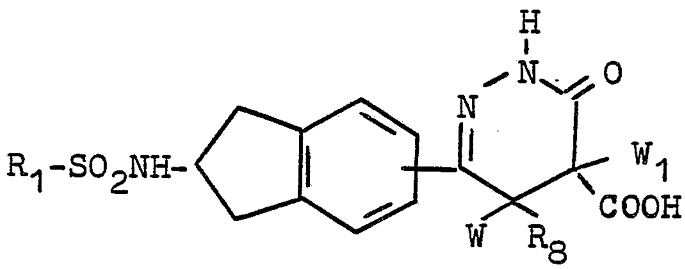

(XII)

in der
$R_1$ wie in den Ansprüchen 1 bis 5 definiert ist,
$R_8$ ein Wasserstoffatom oder eine Alkylgruppe mit 1 bis 3 Kohlenstoffatomen,
W ein Wasserstoffatom und
$W_1$ ein Wasserstoffatom oder eine Alkylgruppe mit 1 bis 3 Kohlenstoffatomen oder W und $W_1$ zusammen eine weitere Bindung bedeuten, decarboxyliert wird oder
l) zur Herstellung von Verbindungen der allgemeinen Formel I, in der $R_2$ einen der in den Ansprüchen 1 bis 5 erwähnten Oxobuttersäureesterreste darstellt, eine Verbindung der allgemeinen Formel

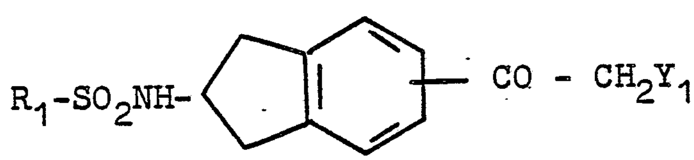

(XIII)

in der
$R_1$ wie in den Ansprüchen 1 bis 5 definiert ist und
$Y_1$ eine nukleophile Austrittsgruppe wie ein Halogenatom, z.B. ein Chlor-, Brom-oder Jodatom, oder $Y_1$ zusammen mit dem benachbarten Wasserstoffatom ein Sauerstoffatom darstellt, mit einer Verbindung der allgemeinen Formel

$R_9$ - CH - $COOR_{11}$
$R_8$
, (XIV)

in der
$R_8$ ein Wasserstoffatom oder eine Alkylgruppe mit 1 bis 3 Kohlenstoffatomen,
$R_9$ ein Wasserstoffatom, eine Alkylgruppe mit 1 bis 3 Kohlenstoffatomen oder eine Alkoxycarbonylgruppe mit insgesamt 2 bis 4 Kohlenstoffatomen, wobei jedoch nur einer der Reste $R_8$ oder $R_9$ eine Alkylgruppe mit 1 bis 3 Kohlenstoffatomen darstellen kann, oder auch, falls $R_9$ eine Alkoxycarbonylgruppe mit insgesamt 2

bis 4 Kohlenstoffatomen darstellt, $R_8$ zusammen mit der CH-Gruppe eine Methylengruppe und $R_{11}$ eine Alkylgruppe mit 1 bis 3 Kohlenstoffatomen darstellen, in Gegenwart einer Base umgesetzt wird und gewünschtenfalls anschließend eine so erhaltene Verbindung der allgemeinen Formel I, in der $R_2$ eine Hydroxycarbonylgruppe darstellt oder enthält, mittels Veresterung in eine entsprechende Alkoxycarbonylverbindung übergeführt wird und/oder
eine so erhaltene Verbindung der allgemeinen Formel I in ihre Enantiomeren aufgetrennt wird und/oder
eine so erhaltene Verbindung der allgemeinen Formel I, falls diese eine Carboxygruppe enthält, in ihre Additionssalze mit anorganischen oder organischen Basen, insbesondere für die pharmazeutische Anwendung in ihre physiologisch verträglichen Additionssalze, übergeführt wird.